# EUROPÄISCHE PATENTSCHRIFT

(11) **EP 1 140 952 B1**
(45) Veröffentlichungstag und Bekanntmachung des Hinweises auf die Patenterteilung: **03.09.2003**
(21) Anmeldenummer: 99968365.9
(22) Anmeldetag: 22.12.1999
(51) Int. Cl.: C07F 9/572, A61K 31/675, A61P 31/00, A61P 33/00, A01N 57/24, C07F 9/59, C07F 9/553, C07F 9/6561, C07F 9/62

(54) **PHOSPHORORGANISCHE VERBINDUNGEN UND IHRE VERWENDUNG**
ORGANO-PHOSPHORUS COMPOUNDS AND THEIR UTILIZATION
COMPOSES ORGANOPHOSPHORIQUES ET LEUR UTILISATION

(30) Priorität: 22.12.1998 DE 19859426
(43) Veröffentlichungstag der Anmeldung: 10.10.2001
(73) Patentinhaber: BioAgency AG, 22525 Hamburg (DE)
(72) Erfinder: JOMAA, Hassan, D-35398 Giessen (DE)
(74) Vertreter: Dreiss, Fuhlendorf, Steimle & Becker
(86) Internationale Anmeldenummer: EP9910274
(87) Internationale Veröffentlichungsnummer: WO00037477

(56) Entgegenhaltungen:
- WO-A-99/52515
- US-A- 4 206 156
- US-A- 4 693 742
- REGITZ M.: "C-Alkylierung von Dimethyl (bzw. Diäthyl)phosphono- und Diphenyl- phosphinyldiazomethan mit Carbonylverbindungen (1)" TETRAHEDRON LETTERS., Nr. 38, 1972, Seiten 3979-3982, XP002136657 ELSEVIER SCIENCE PUBLISHERS, AMSTERDAM., NL ISSN: 0040-4039
- CHEMICAL ABSTRACTS, vol. 093, no. 19, 10. November 1989 (1989-11-10) Columbus, Ohio, US; abstract no. 186456, KAMIYA T ET AL: "Studies on new phosphonic acid-containing antibiotics: synthesis of FR-31564 and related antibiotics" XP002122512 & CURR. CHEMOTHER. INFECT. DIS., PROC. INT. CONGR. CHEMOTHER., 11TH (43MKAT);1980; VOL.1,; PP.355-8, Fujisawa Pharm. Co., Ltd.;Res. Lab.; Osaka; Japan
- H C NEU ET AL: "In Vitro and In Vivo Antibacterial Activity of FR-31564, a Phosphonic Acid Antimicrobial Agent" ANTIMICROBIAL AGENTS AND CHEMOTHERAPY,US,AMERICAN SOCIETY FOR MICROBIOLOGY, WASHINGTON, DC, Bd. 19, Nr. 6, Juni 1981 (1981-06), Seiten 1013-1023-1023, XP002113260 ISSN: 0066-4804
- CHEMICAL ABSTRACTS, vol. 105, no. 19, 10. November 1986 (1986-11-10) Columbus, Ohio, US; abstract no. 166897, YAMAJI T ET AL: "N-Substituted alkyl amine phosphates as herbicides" XP002122513 & JP 61 106504 A (TEIJIN LTD.;JAPAN)

## Beschreibung

Die Erfindung betrifft phosphororganische Verbindungen, ihre Salze, Ester und Amide und ihre Verwendung zur therapeutischen und prophylaktischen Behandlung von Infektionen bei Mensch und Tier, die durch Viren, Bakterien, Pilze und Parasiten hervorgerufen werden, und ihre Verwendung als Fungizid, Bakterizid und Herbizid bei Pflanzen. Erfindungsgemäß umfassen die phosphororganischen Verbindungen Phosphinoylderivate, Phosphinsäurederivate und Phosphonsäurederivate.

In Tetrahedron letters, Nr. 38, 1972, Seiten 3979-3982 sind 3-Hydroxy-2-oxo-4-dimethylphosphono- und 3-Hydroxy-2-oxo-4-diphenylphosphinyl-1,2-dihydrochinoline und ihre Herstellung beschrieben.

In Chemical Abstracts, Vol. 093, No. 19, 10. November 1989 & Curr. Chemother. Infect, Dis., Proc. Int. Congr. Chemother., 11^{th}; 1980; Vol,1; Seiten 355-8, in Chemical Abstracts, Vol. 105, No. 19, 10. November 1986 & JP 61 106504 A, in US-A- 4206 156, in US-A- 4 693 742 und in WO 99 525 15 A sind N-substituierte Alkylaminphosphate beschrieben. Diese Verbindungen werden als geeignet zur Behandlung von bakteriellen Infektionen beschrieben. In WO 99 525 15 A wird ferner ihre Verwendung bei viralen, fungiziden und parasitären Infektionen beschrieben.

Es besteht ein starker Bedarf, für die Bereicherung der Behandlung von Mensch und Tier Mittel bereitzustellen, die nicht nur eine starke Wirksamkeit besitzen, sondern auch im Gegensatz zu anderen Arzneimitteln verringerte Nebenwirkungen zeigen und damit eine geringere Gesundheitsgefahr für den Menschen bedeuten.

Aufgabe der vorliegenden Erfindung ist es daher, eine Substanz bereitzustellen, die universell bei Infektionen durch Viren, Bakterien, Pilze und Parasiten bei Menschen und Tieren einsetzbar ist und die oben angegebenen Bedingungen erfüllt.

Diese Aufgabe wird in völlig überraschender Weise durch die in Anspruch 1 definierte Stoffgruppe gelöst. Diese Stoffgruppe zeigt eine antünfektiöse Wirkung gegen Viren, Bakterien, Pilze und ein- und mehrzellige Parasiten. Ferner wurde auch eine fungizide, bakterizide und herbizide Wirkung bei Pflanzen Festgestellt.

Die erfindungsgemäßen phosphororganischen verbindungen entsprechen der allgemeinen Formel (I): 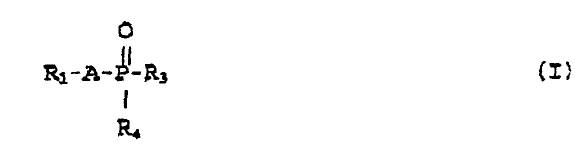 wobei A aus der Gruppe ausgewählt ist, die aus einem (C₁₋₉)-Alkylenrest, der ein oder mehrere Doppelbindungen aufweisen kann und mit Hydroxy-, Halogen-, Amino-, Oxogruppen mit verzweigten oder unverzweigten C₁₋₉-Alkylgruppen und C₂₋₉-Alkenyl-gruppen substituiert sein kann, wobei die C₁₋₉-Alkylgruppen und C₂₋₉-Alkenylgruppen mit Wasserstoff-, Hydroxy-, Amino-, Halogen- und Oxogruppen substituiert sein können, -C-O-C- und -C-N-C- besteht,
wobei die Kohlenstoffatome von -C-O-C- und -C-N-C- mit einem Alkyl mit bis zu 7 Kohlenstoffatomen oder Hydroxygruppen substituiert sein können,
oder in der A der folgenden Formel (II) entspricht: 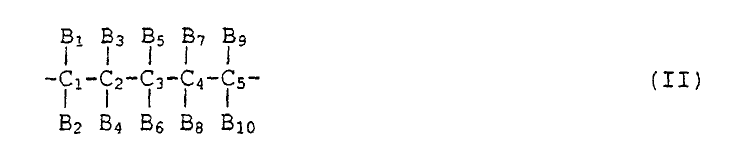 wobei ein oder mehrere der Kohlenstoffatome, ausgewählt aus der Gruppe C₃, C₄, C₅, mitsamt ihren Substituenten auch wegfallen können, und mindestens ein vorliegender Substituent von B₁ bis B₁₀ eine C₃₋₈-Cycloalkyl-(C₀₋₉)-alkylgruppe ist, wobei sowohl die C₃₋₈-Cycloalkylgruppe als auch die C₀₋₉-Alkylgruppe ein oder mehrere Doppelbindungen aufweisen können und ein oder zwei Kohlenstoffatome der Cycloalkylgruppe durch Stickstoff-, Sauerstoff- oder Schwefelatome ersetzt sein können, und wobei sowohl die Cycloalkylgruppe als auch die Alkylgruppe mit Hydroxy-, Halogen-, Amino-, Oxogruppen mit verzweigten oder unverzweigten C₁₋₉-Alkylgruppen und C₂₋₉-Alkenylgruppen substituiert sein können, wobei die C₁₋₉-Alkylgruppen und C₂₋₉-Alkenylgruppen mit Wasserstoff-, Hydroxy-, Amino-, Halogen- und Oxogruppen substituiert sein können, und die restlichen vorliegenden Substituenten B₁ bis B₁₀ aus der Gruppe ausgewählt sind, die aus Wasserstoff, Hydroxy-, Halogen-, Aminogruppen. C₁₋₂₆-Alkylresten, C₁₋₂₆-Alkoxyresten, C₁₋₂₆-Alkoxy-C₁₋₂₆-Alkylresten besteht oder beide Substituenten eines C-Atoms zusammen eine Oxogruppe bilden, wobei jeder C₁₋₂₆-Alkylrest und jeder C₁₋₂₆-Alkoxyrest verzweigt oder unverzweigt und gesättigt oder mit ein oder mehreren Doppelbindungen ungesättigt sein kann und mit Hydroxy-, Amino-, Halogenund Oxogruppen substituiert sein kann,
in der R₁ aus der Gruppe ausgewählt ist, die aus 5- und 6-gliedrigen Heterocyclen mit mindestens einem Stickstoffatom im Ring oder einem polycyclischen Kohlenstoff mit mindestens einem dieser Heterocyclen besteht, wobei mindestens eines dieser Stickstoffatome zu einer Hydroxamsäuregruppe oder Hydroxamsäureestergruppe gehört, und der Heterocyclus gesättigt oder mit ein oder mehreren Doppel- oder Dreifachbindungen ungesättigt und damit auch aromatisch sein kann und mit Hydroxy-, Halogen-, Amino-, Oxogruppen und mit verzweigten oder unverzweigten C₁₋₉-Alkylgruppen und C₂₋₉-Alkenylgruppen substituiert sein kann, wobei die C₁₋₉-Alkylgruppen und C₂₋₉-Alkenylgruppen gesättigt oder mit ein oder mehreren Doppeloder Dreifachbindungen ungesättigt sein können und mit Wasserstoff-, Hydroxy-, Amino-, Halogen- und Oxogruppen substituiert sein können, wobei das Stickstoffatom der Hydroxamsäuregruppe oder -säureestergruppe mit OR₅ substiuiert ist und

R₅ aus der Gruppe ausgewählt ist, die aus Wasserstoff, substituiertem und unsubstituiertem C₁₋₉-Alkyl, substituiertem und unsubstituiertem Hydroxy-C₁₋₉-alkyl, substituiertem und unsubstituiertem C₂₋₉-Alkenyl, substituiertem und unsubstituiertem C₂₋₉-Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest besteht,
in der R₃ und R₄ gleich oder verschieden sind und aus der Gruppe ausgewählt sind, die aus Wasserstoff, substituiertem und unsubstituiertem C₁₋₂₆-Alkyl, Hydroxy-C₁₋₂₆-alkyl, substituiertem und unsubstituiertem Aryl, substituiertem und unsubstituiertem Acyl, substituiertem und unsubstituiertem Aralkyl, substituiertem und unsubstituiertem C₂₋₂₆-Alkenyl, substituiertem und unsubstituiertem C₂₋₂₆-Alkinyl, substituiertem und unsubstituiertem Cycloalkyl, substituiertem und unsubstituiertem heterocyclischem Rest, Halogen, OX₃ und OX₄ besteht,
wobei X₃ und X₄ gleich oder verschieden sind und aus der Gruppe ausgewählt sind, die aus Wasserstoff, substituiertem und unsubstituiertem C₁₋₂₆-Alkyl, substituiertem und unsubstituiertem Hydroxy-C₁₋₂₆-alkyl, substituiertem und unsubstituiertem Aryl, substituiertem und unsubstituiertem Aralkyl, substituiertem und unsubstituiertem C₂₋₂₆-Alkenyl, substituiertem und unsubstituiertem C₂₋₂₆-Alkinyl, substituiertem und unsubstituiertem Cycloalkyl, substituiertem und unsubstituiertem heterocyclischem Rest, einem Silyl, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht,
und deren pharmazeutisch akzeptablen Salze, Ester und Amide und Salze der Ester.

Kommen mehrere Heteroatome in dem Heterocyclus R₁ vor, so können auch Sauer- oder Schwefelatome im Ring vorliegen.

Bevorzugt entsprechen die phosphororganische Verbindungen der Formel (III) 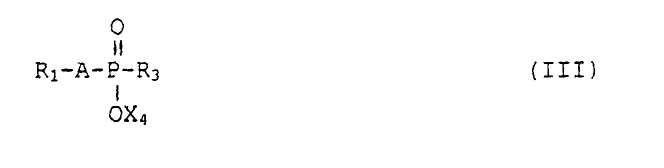 wobei R₃ bevorzugt Wasserstoff, Methyl, Ethyl, ein Amidrest oder OX₃ ist und X₄ aus der Gruppe ausgewählt ist, die aus Wasserstoff, Natrium, Kalium, Methyl, Ethyl besteht, wobei X₃ wie oben definiert ist
und besonders bevorzugt der Formel (IV) 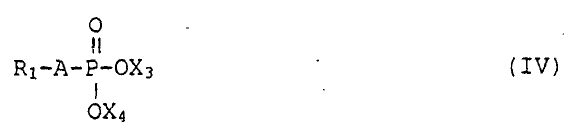 wobei X₃ und X₄ gleich oder verschieden und aus der Gruppe ausgewählt sind, die aus Wasserstoff, einem (C₁₋₃)-Alkyl, einem Metall der ersten, zweiten oder dritten Hauptgruppe des Periodensystems, Ammonium, substituiertem Ammonium, oder Ammoniumverbindungen, die sich von Ethylendiamin oder Aminosäuren ableiten, besteht.

Bevorzugt sind X₃ und X₄ ein Metall der ersten, zweiten oder dritten Hauptgruppe des Periodensystems, Ammonium, substituiertes Ammonium, oder Ammoniumverbindungen, die sich von Ethylendiamin oder Aminosäuren ableiten. D.h. es werden die Salzverbindungen der phosphororganischen Verbindungen mit organischen oder anorganischen Basen (z.B. Natriumsalz, Kaliumsalz, Calciumsalz, Aluminiumsalz, Ammoniumsalz, Magnesiumsalz, Triethylaminsalz, Ethanolaminsalz, Dicyclohexylaminsalz, Ethylendiaminsalz, N,N'-Dibenzylethylen-diaminsalz etc.) sowie Salze mit Aminosäuren (z.B. Arginin-salz, Asparaginsäuresalz, Glutaminsäuresalz etc.) und dergleichen gebildet.

Besonders bevorzugt sind X₃ und X₄ gleich oder verschieden und aus der Gruppe ausgewählt, die aus Wasserstoff, Natrium, Kalium, Methyl, Ethyl besteht.

Bevorzugt ist A aus der Gruppe ausgewählt, die aus Alkylen, Alkenylen, Hydroxyalkylen und Oxoalkylen besteht.

Bevorzugt ist A so gewählt, daß zwischen dem Phosphoratom und dem Stickstoffatom des Heterocyclus eine dreigliedrige Verbindungskette gebildet wird. A ist zum Beispiel vorteilhaft ein Methylen, Hydroxymethylen, Ethylen, Ethenylen, Hydroxyethylen und kann insbesondere auch mit einer Oxogruppe substituiert sein.

Bevorzugt verbindet auch die Kohlenstoffkette von A mit der Formel (II) zusammen mit den Atomen des Heterocyclus das Stickstoff und das Phorphoratom über drei Atome. Ist das in α-Stellung zum Stickstoff- oder Phosphoratom angeordnete Kohlenstoffatom mit einer Oxogruppe in der Verbindungskette substituiert, so sind auch Verbindungsketten mit vier Kohlenstoffatomen bevorzugt. Liegen sowohl eine Oxogruppe in α-Stellung zum Stickstoffatom als auch eine weitere Oxogruppe in α-Stellung zum Phosphoratom in dieser Verbindungskette vor, so ist auch eine Verbindungskette mit fünf Kohlenstoffatomen bevorzugt. Ist das in α-Stellung zum Phosphoratom angeordnete Kohlenstoffatom mit einer Hydroxygruppe substituiert, so sind Verbindungsketten mit vier Kohlenstoffatomen bevorzugt. In diesem Fall sind für R₃ und R₄ auch Methylengruppen bevorzugt.

Im folgenden werden ganz besonders geeignete Verbindungen aufgeführt: 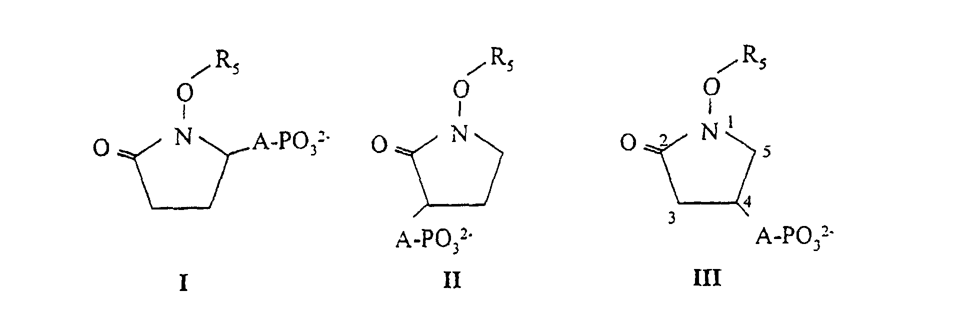 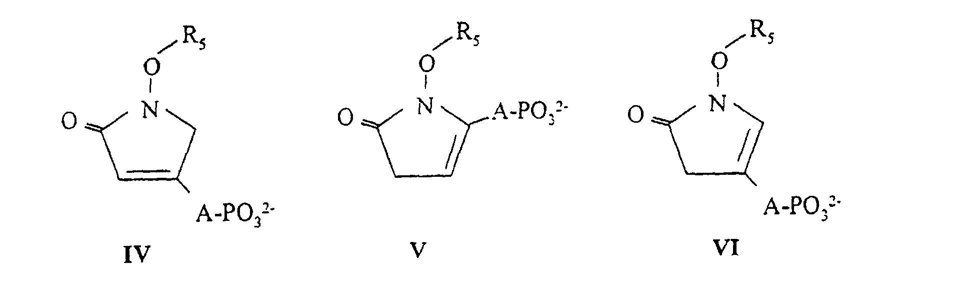 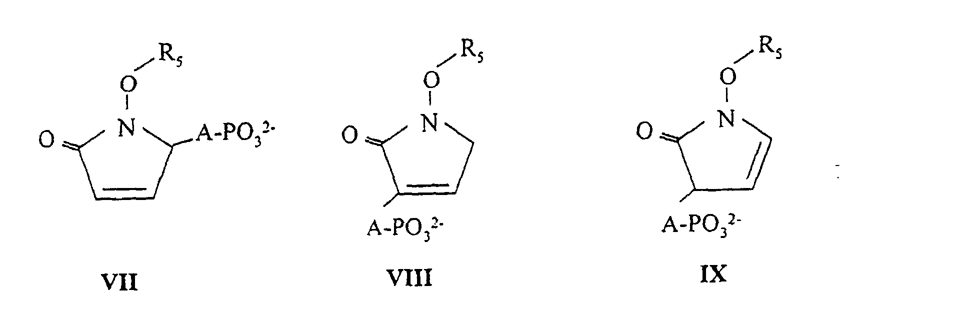 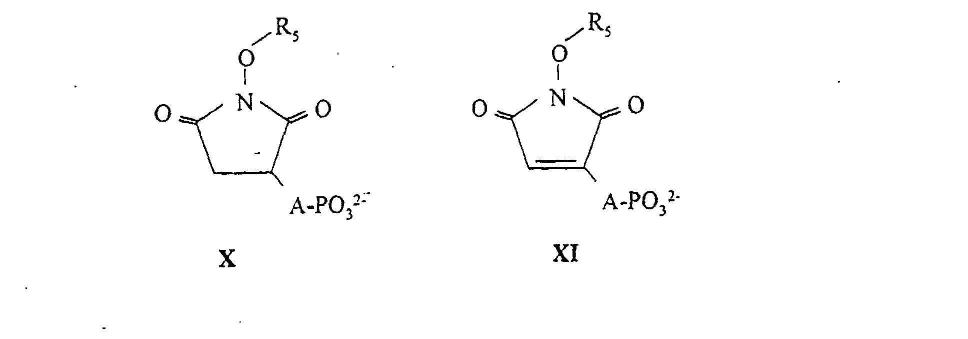 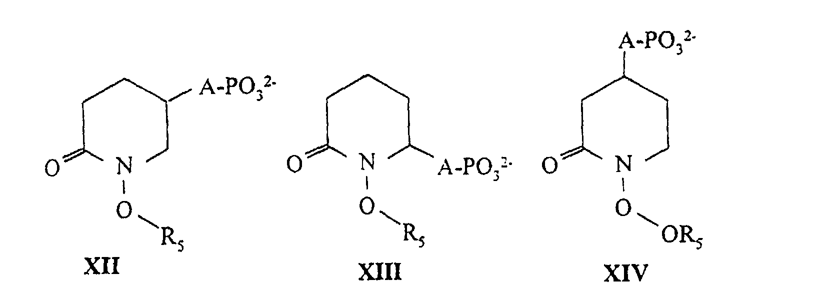 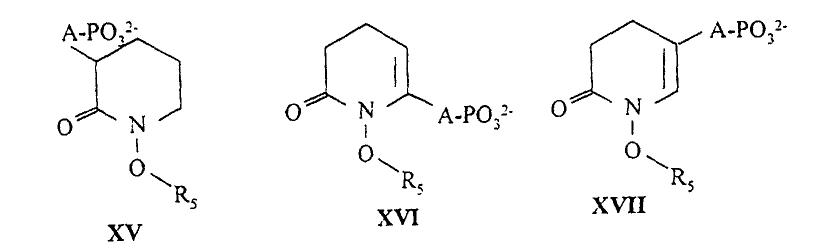 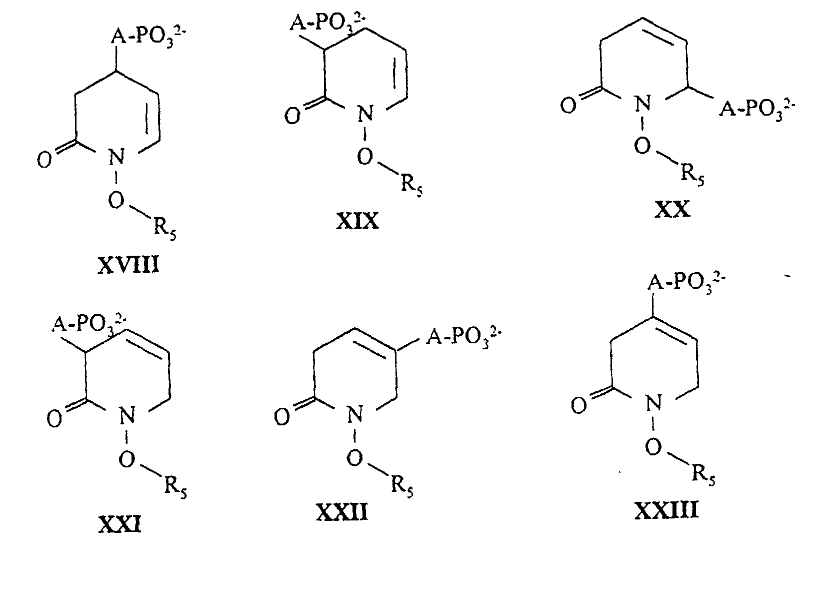 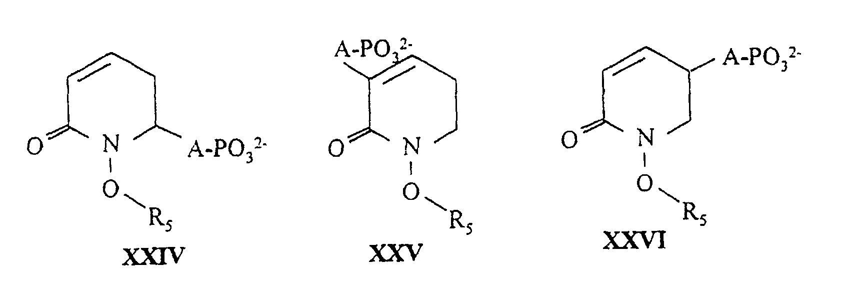 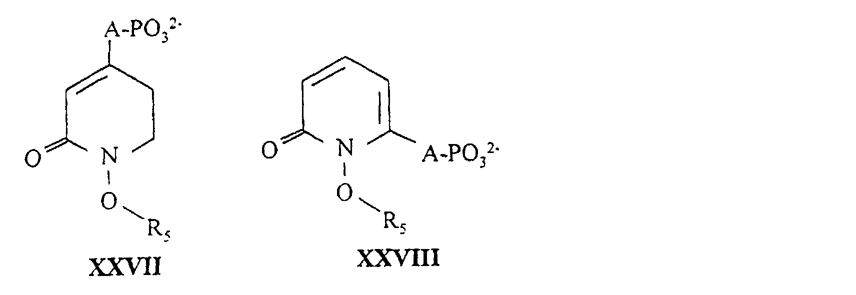 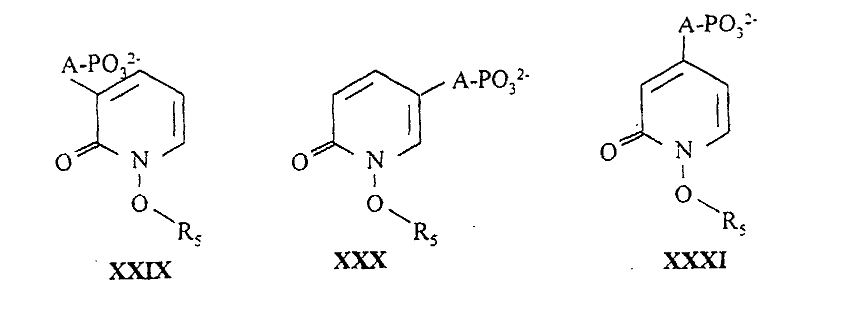 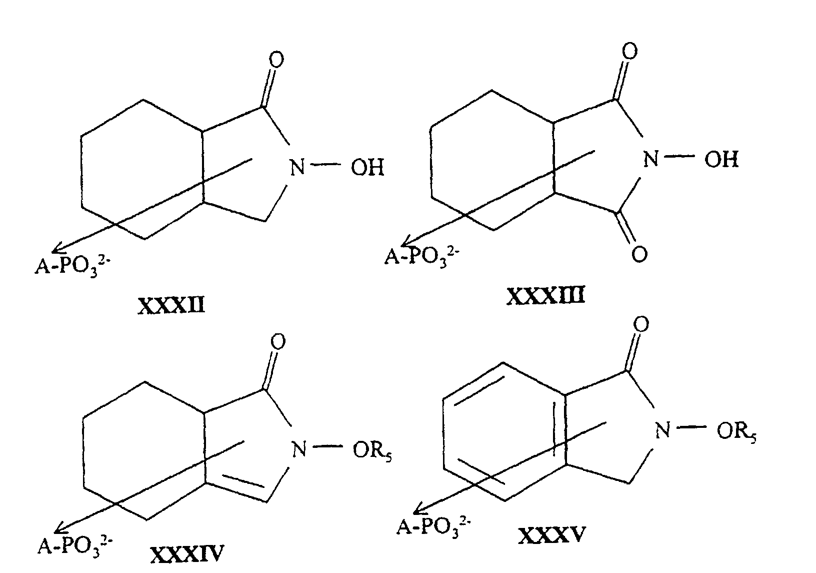 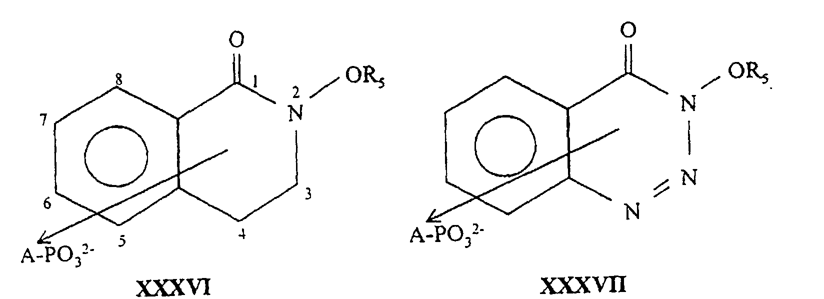 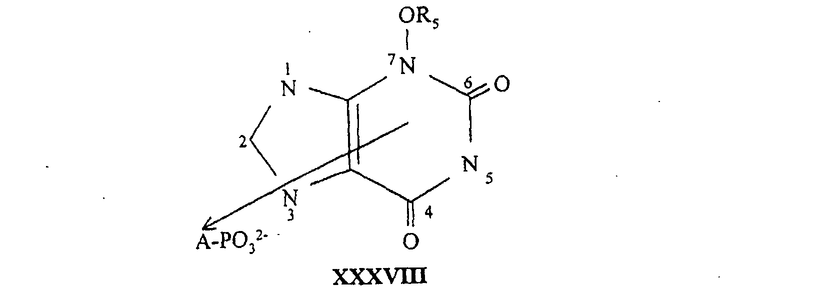 sowie die entsprechenden Phosphinsäure- und Phosphinoylderivate, wobei R₅ wie in Formel (I) definiert ist.

Besonderheiten der obigen Definitionen und geeignete Beispiele dafür werden nachfolgend angegeben:
"Acyl" ist ein Substituent, der von einer Säure stammt, wie von einer organischen Carbonsäure, Kohlensäure. Carbaminsäure oder der den einzelnen vorstehenden Säuren entsprechenden Thiosäure oder Imidsäure, oder von einer organischen Sulfonsäure, wobei diese Säuren jeweils aliphatische, aromatische und/oder heterocyclische Gruppen im Molekül umfassen sowie Carbamoyl oder Carbamimidoyl.

Geeignete Beispiele für diese Acylgruppen werden nachfolgend angegeben. Als aliphatische Acylgruppen werden von einer aliphatischen Säure stammende Acylreste bezeichnet, zu denen die folgenden gehören:
Alkanoyl (z.B. Formyl, Acetyl, Propionyl, Butyryl, Isobutyryl, Valeryl, Isovaleryl, Pivaloyl etc.);Alkenoyl (z. B. Acryloyl, Methacryloyl, Crotonoyl etc.); Alkylthioalkanoyl (z.B. Methylthioacetyl, Ethylthioacetyl etc.); Alkansulfonyl (z.B. Mesyl, Ethansulfonyl, Propansulfonyl etc.); Alkoxycarbonyl (z.B. Methoxycarbonyl, Ethoxycarbonyl, Propoxycarbonyl, Isopropoxycarbonyl, Butoxycarbonyl, Isobutoxycarbonyl etc.); Alkylcarbamoyl (z.B. Methylcarbamoyl etc.); (N-Alkyl)-thiocarbamoyl (z.B. (N-Methyl)-thiocarbamoyl etc.); Alkylcarbamimidoyl (z.B. Methylcarbamimidoyl etc.); Oxalo; Alkoxalyl (z.B. Methoxalyl, Ethoxalyl, Propoxalyl etc.).

Bei den obigen Beispielen für aliphatische Acylgruppen kann der aliphatische Kohlenwasserstoffteil, insbesondere die Alkylgruppe bzw. der Alkanrest. ggf. einen oder mehrere geeignete Substituenten aufweisen, wie Amino, Halogen (z.B. Fluor. Chlor, Brom etc.), Hydroxy, Hydroxyimino, Carboxy, Alkoxy (z.B. Methoxy, Ethoxy, Propoxy etc.), Alkoxycarbonyl, Acylamino (z.B. Benzyloxycarbonylamino etc.), Acyloxy (z.B. Acetoxy, Benzoyloxy etc.) und dergleichen; als bevorzugte aliphatische Acylreste mit solchen Substituenten sind z.B. mit Amino, Carboxy, Amino und Carboxy, Halogen, Acylamino oder dergleichen substituierte Alkanoyle zu nennen.

Als aromatische Acylreste werden solche Acylreste bezeichnet, die von einer Säure mit substituierter oder nicht substituierter Arylgruppe stammen, wobei die Arylgruppe Phenyl, Toluyl, Xylyl, Naphthyl und dergleichen umfassen kann; geeignete Beispiele werden nachfolgend angegeben:
Aroyl (z.B. Benzoyl, Toluoyl, Xyloyl, Naphthoyl, Phthaloyl etc.); Aralkanoyl (z.B. Phenylacetyl etc.); Aralkenoyl (z.B. Cinnamoyl etc.); Aryloxyalkanoyl (z.B. Phenoxyacetyl etc.); Arylthioalkanoyl (z.B. Phenylthioacetyl etc.); Arylaminoalkanoyl (z.B. N-Phenylglycyl, etc.); Arensulfonyl (z.B.Benzolsulfonyl, Tosyl bzw. Toluolsulfonyl, Naphthalinsulfonyl etc.); Aryloxycarbonyl (z.B. Phenoxycarbonyl, Naphthyl-oxycarbonyl etc.); Aralkoxycarbonyl (z.B. Benzyloxycarbonyl etc.); Arylcarbamoyl (z.B. Phenylcarbamoyl, Naphthylcarbamoyl etc.); Arylglyoxyloyl (z.B. Phenylglyoxyloyl etc.).

Bei den vorstehenden Beispielen für aromatische Acylreste kann der aromatische Kohlenwasserstoffteil (insbesondere der Arylrest) und/oder der aliphatische Kohlenwasserstoffteil (insbesondere der Alkanrest) ggf. einen oder mehrere geeignete Substituenten aufweisen, wie solche, die als geeignete Substituenten für die Alkylgruppe bzw. den Alkanrest bereits angegeben wurden. Insbesondere und als Beispiel für bevorzugte aromatische Acylreste mit besonderen Substituenten werden mit Halogen und Hydroxy oder mit Halogen und Acyloxy substituiertes Aroyl und mit Hydroxy, Hydroxyimino, Dihalogenalkanoyloxyimino substituiertes Aralkanoyl angegeben sowie Arylthiocarbamoyl (z.B. Phenylthiocarbamoyl etc.); Arylcarbamimidoyl (z.B. Phenylcarbamimidoyl etc.).

Als heterocyclischer Acylrest wird ein Acylrest verstanden, der von einer Säure mit heterocyclischer Gruppe stammt: dazu gehören:
Heterocyclisches Carbonyl, bei dem der heterocyclische Rest ein aromatischer oder aliphatischer 5-bis 6-gliedriger Heterocyclus mit zumindest einem Heteroatom aus der Gruppe Stickstoff, Sauerstoff und Schwefel ist (z.B. Thiophenyl, Furoyl, Pyrrolcarbonyl, Nicotinoyl etc.);
Heterocyclus-Alkanoyl, bei dem der heterocyclische Rest 5- bis 6-gliedrig ist und zumindest ein Heteroatom aus der Gruppe Stickstoff, Sauerstoff und Schwefel aufweist (z.B. Thiophenyl-acetyl, Furylacetyl, Imidazolylpropionyl, Tetrazolylacetyl, 2-(2-Amino-4-thiazolyl)-2-methoxyiminoacetyl etc.) und dergleichen.

Bei den obigen Beispielen für heterocyclische Acylreste kann der Heterocyclus und/oder der aliphatische Kohlenwasserstoffteil ggf. einen oder mehrere geeignete Substituenten aufweisen, wie die gleichen, die als geeignet für Alkyl- und Alkangruppen angegeben wurden.

"Alkyl" ist ein gerad- oder verzweigtkettiger Alkylrest, wie Methyl, Ethyl, Propyl, Isopropyl, Butyl, Isobutyl, tert.-Butyl, Pentyl, Hexyl und dergleichen.

"Hydroxylalkyl" ist ein gerad- oder verzweigtkettiger Alkylrest, der mindestens eine Hydroxylgruppe aufweist, bevorzugt ein oder zwei Hydroxylgruppen.

Zu "Alkenyl" gehören gerad- oder verzweigtkettige Alkenylgruppen, wie z.B. Vinyl, Propenyl (z.B. 1-Propenyl, 2-Propenyl), 1-Methylpropenyl, 2-Methylpropenyl, Butenyl, 2-Ethylpropenyl, Pentenyl, Hexenyl.

Zu "Alkinyl" gehören gerad- oder verzweigtkettige Alkinylgruppen.

Cycloalkyl steht vorzugsweise für ein ggfs. substituiertes C₃₋₈-Cycloalkyl; als mögliche Substituenten sind u.a. Alkyl, Alkenyl, Alkinyl, Alkoxy (z.B. Methoxy, Ethoxy etc.), Halogen (z.B. Fluor, Chlor, Brom etc.), Nitro und dergleichen geeignet.

Aryl ist ein aromatischer Kohlenwasserstoffrest, wie Phenyl Naphthyl usw., der ggf. einen oder mehrere geeignete Substituenten aufweisen kann, wie Alkyl, Alkenyl, Alkinyl, Alkoxy (z.B. Methoxy, Ethoxy etc.), Halogen (z.B. Fluor, Chlor, Brom etc.), Nitro und dergleichen.

Zu "Aralkyl" gehören Mono-, Di-, Triphenylalkyle wie Benzyl, Phenethyl, Benzhydryl, Trityl und dergleichen, wobei der aromatische Teil ggf. ein oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Vorzugsweise können die Reste X₃ und X₄ so gewählt werden, daß Ester an der Phosphinogruppe oder Phosphonogruppe gebildet werden. Zu geeigneten Beispielen für solche Ester gemäß der Formeln (I), (III) und (IV) zählen geeignete Mono- und Diester, und zu bevorzugten Beispielen für solche Ester gehören Alkylester (z.B. Hexadecanylester, Octadecanylester etc.); Aralkylester (Benzylester, Phenethylester, Benzhydrylester, Tritylester etc.); Arylester (z.B. Phenylester, Tolylester, Naphthylester etc.); Aroylalkylester (z.B. Phenacylester etc.); und Silylester (z.B. von Trialkylhalogensilyl, Dialkyldihalogensilyl, Alkyltrihalogensilyl, Dialkylarylhalogensilyl, Trialkoxyhalogensilyl, Dialkylaralkylhalogensilyl, Dialkoxydihalogensilyl, Trialkoxyhalogensilyl etc.) und dergleichen.

Beim obigen Ester kann der Alkan- und/oder Arenteil wahlweise zumindest einen geeigneten Substituenten aufweisen wie Halogen, Alkoxy, Hydroxy, Nitro oder dergleichen.

Die erfindungsgemäß verwendeten Verbindungen gemäß der Formeln (I), (III) und (IV) können in ihrer protonierten Form als Ammoniumsalz organischer oder anorganischer Säuren, wie Salzsäure, Bromwasserstoffsäure, Schwefelsäure, Salpetersäure, Methansulfonsäure, p-Toluolsulfonsäure, Essigsäure, Milchsäure, Maleinsäure, Fumarsäure, Oxalsäure, Weinsäure, Benzoesäure, etc. vorliegen.

Die erfindungsgemäß verwendeten Verbindungen der Formeln (I), (III) und (IV) lassen beispielsweise für Doppelbindungen enthaltende oder chirale Gruppen R₁, R₃, R₄, X₃, X₄ oder A das Auftreten räumlicher Isomerer zu. Die erfindungsgemäße Verwendung der Verbindungen umfaßt alle räumlichen Isomere sowohl als Reinstoffe als auch in Form ihrer Mischungen.

Die phosphororganischen Verbindungen sind für die therapeutische und prophylaktische Behandlung von Infektionen bei Mensch und Tier geeignet, die durch Viren, Bakterien, ein- und mehrzellige Parasiten und Pilze hervorgerufen werden.

Die Verbindungen sind gegen einzellige Parasiten (Protozoen) wirksam, insbesondere gegen Erreger der Malaria und der Schlafkrankheit sowie der Chagas-Krankheit, der Toxoplasmose. der Amöbenruhr, der Leishmaniosen, der Trichomoniasis. der Pneumozystose, der Balantidiose, der Kryptosporidiose, der Sarkozystose, der Akanthamöbose, der Naeglerose, der Kokzidiose, der Giardiose und der Lambliose.

Sie sind daher insbesondere als Malariaprophylaxe und als Prophylaxe der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose geeignet.

Die erfindungsgemäßen Wirkstoffe sind insbesondere gegen die folgenden Bakterien einsetzbar:
Bakterien der Familie Propionibacteriaceae, insbesondere der Gattung Propionibacterium, insbesondere die Art Propionibacterium acnes, Bakterien der Familie Actinomycetaceae, insbesondere der Gattung Actinomyces, Bakterien der Gattung Corynebacterium, insbesondere die Arten Corynebacterium diphteriae und Corynebacterium pseudotuberculosis, Bakterien der Familie Mycobacteriaceae, der Gattung Mycobacterium, insbesondere die Arten Mycobacterium leprae. Mycobacterium tuberculosis, Mycobacterium bovis und Mycobacterium avium, Bakterien der Familie Chlamydiaceae, insbesondere die Spezies Chlamydia trachomatis und Chlamydia psittaci. Bakterien der Gattung Listeria, insbesondere die Art Listeria monocytogenes, Bakterien der Art Erysipelthrix rhusiopathiae, Bakterien der Gattung Clostridium, Bakterien der Gattung Yersinia, der Spezies Yersinia pestis, Yersinia pseudotuberculosis. Yersinia enterocolitica und Yersinia ruckeri, Bakterien der Familie Mycoplasmataceae, der Gattungen Mycoplasma und Ureaplasma, insbesondere die Art Mycoplasma pneumoniae, Bakterien der Gattung Brucella, Bakterien der Gattung Bordetella, Bakterien der Familie Neisseriaceae, insbesondere der-Gattungen Neisseria und Moraxella, insbesondere die Arten Neisseria meningitides, Neisseria gonorrhoeae und Moraxella bovis, Bakterien der Familie Vibrionaceae, insbesondere der Gattungen Vibrio, Aeromonas, Plesiomonas und Photobacterium, insbesondere die Arten Vibrio cholerae, Vibrio anguillarum und Aeromonas salmonicidas, Bakterien der Gattung Campylobacter, insbesondere die Arten Campylobacter jejuni, Campylobacter coli und Campylobacter fetus, Bakterien der Gattung Helicobacter, insbesondere die Art Helicobacter pylori, Bakterien der Familien Spirochaetaceae und der Leptospiraceae, insbesondere der Gattungen Treponema, Borrelia und Leptospira, insbesondere Borrelia burgdorferi, Bakterien der Gattung Actinobacillus, Bakterien der Familie Legionellaceae, der Gattung Legionella, Bakterien der Familie Rickettsiaceae und Familie Bartonellaceae, Bakterien der Gattungen Nocardia und Rhodococcus, Bakterien der Gattung Dermatophilus, Bakterien der Familie Pseudomonadaceae, insbesondere der Gattungen Pseudomonas und Xanthomonas. Bakterien der Familie Enterobacteriaceae, insbesondere der Gattungen Escherichia, Klebsiella, Proteus, Providencia, Salmonella, Serratia und Shigella, Bakterien der Familie Pasteurellaceae, insbesondere der Gattung Haemophilus, Bakterien der Familie Micrococcaceae, insbesondere der Gattungen Micrococcus und Staphylococcus, Bakterien der Familie Streptococcaceae. insbesondere der Gattungen Streptococcus und Enterococcus und Bakterien der Familie Bacillaceae, insbesondere der Gattungen Bacillus und Clostridium.

Damit eignen sich die phosphororganischen Verbindungen und ihre Derivate zur Behandlung der Diphterie, der Acne vulgaris, der Listeriosen, des Rotlaufs bei Tieren, der Gasbrand beim Mensch und beim Tier, Pararauschbrand bei Mensch und Tier, Tuberkulose bei Mensch und Tier. Lepra, und weitere Mykobacteriosen bei Mensch und Tier, der Paratuberkulose der Tiere, Pest. mesenterialen Lymphadenitis und Pseudotuberkulose bei Mensch und Tier, Cholera, Legionärskrankheit, Borreliose bei Mensch und Tier, Leptospirosen bei Mensch und Tier, Syphilis. Campylobacter-Enteritiden bei Mensch und Tier, Moraxella-Keratokonjunc-tivitis und Serositis der Tiere, Brucellosen der Tiere und des Menschen, Milzbrand bei Mensch und Tier, Aktinomykose bei Mensch und Tier, Streptotrichosen, Psittakose/Ornithose bei Tieren, Q-Fieber, Ehrlichiose.

Weiter ist der Einsatz besonders bei der Helicobacter-Eradikationstherapie bei Ulcera des Magendarmtraktes bevorzugt.

Es können auch Kombinationen mit einem weiteren Antibiotikum zur Behandlung der obengenannten Erkrankungen eingesetzt werden. Für Kombinationspräparate mit anderen Antiinfektiva eignen sich insbesondere Isoniazid, Rifampicin, Ethambutol, Pyrazinamid, Streptomycin, Protionamid und Dapson zur Behandlung der Tuberkulose.

Die erfindungsgemäßen Wirkstoffe sind ferner insbesondere bei Infektionen mit folgenden Viren einsetzbar:
Parvoviridae: Parvoviren, Dependoviren, Densoviren, Adenoviridae: Adenoviren, Mastadenoviren, Aviadenoviren, Papovaviridae: Papovaviren, insbesondere Papillomaviren (sogenannte Warzenviren), Polyomaviren, insbesondere JC-Virus, BK-Virus, und Miopapovaviren, Herpesviridae: Alle Herpesviren, insbesondere Herpes-Simplex-Viren, der Varizellen/Zoster-Viren, menschlicher Zytomegalievirus, Epstein-Barr-Viren, alle humanen Herpesviren, humanes Herpesvirus 6, Humanes Herpesvirus 7, humanes Herpesvirus 8, Poxviridae:Pockenviren, Orthopox-, Parapox-, Molluscum-Contagiosum-Virus, Aviviren, Capriviren, Leporipoxviren, alle primär hepatotropen Viren, Hepatitisviren: Hepatitis-A-Viren, Hepatitis-B-Viren, Hepatitis-C-Viren, Hepatitis-D-Viren, Hepatitis-E-Viren, Hepatitis-F-Viren, Hepatits-G-Viren, Hepadnaviren: sämtliche Hepatitisviren, Hepatitis-B-Virus, Hepatitis-D-Viren, Picornaviridae: Picornaviren, alle Enteroviren, alle Polioviren, alle Coxsackieviren, alle Echoviren, alle Rhinoviren. Hepatitis-A-Virus, Aphthoviren, Calciviridae: Hepatitis-E-Viren, Reoviridae: Reoviren, Orbiviren. Rotaviren, Togaviridae: Togaviren, Alphaviren, Rubiviren, Pestiviren, Rubellavirus, Flaviviridae: Flaviviren, FSME-Virus, Hepatitis-C-Virus, Orthomyxoviridae: Alle Influenzaviren, Paramyxoviridae: Paramyxoviren, Morbillivirus, Pneumovirus, Masernvirus. Mumpsvirus, Rhabdoviridae: Rhabdoviren, Rabiesvirus, Lyssavirus, viskuläres Stomatitisvirus, Coronaviridae: Coronaviren. Bunyaviridae: Bunyaviren, Nairovirus. Phlebovirus. Uukuvirus, Hantavirus, Hantaanvirus, Arenaviridae: Arenaviren, lymphozytäres Choriomeningitis-Virus, Retroviridae: Retroviren, alle HTL-Viren, humanes T-cell Leukämievirus. Oncornaviren, Spumaviren, Lentiviren, alle HI-Viren, Filoviridae: Marburg- und Ebolavirus. Slow-virus-Infektionen, Prionen, Onkoviren und Leukämieviren.

Die erfindungsgemäßen phosphororganischen Verbindungen sind somit zur Bekämpfung folgender viraler Infekte geeignet:
Eradikation von Papillomaviren zur Vorbeugung von Tumoren, insbesondere von Tumoren der Geschlechtsorgane verursacht durch Papillomaviren beim Menschen, Eradikation von JC-Viren und BK-Viren, Eradikation von Herpesviren, Eradikation humaner Herpesviren 8 zur Behandlung der Kaposi-Sarkoma, Eradikation von Zytomegalie-Viren vor Transplantationen, Eradikation von Eppstein-Barr-Viren vor Transplantation und zur Vorbeugung von Eppstein-Barr-Viren-assozierten Tumoren, Eradikation von Hepatitisviren zur Behandlung von chronischen Leber-Erkrankungen und zur Vorbeugung von Lebertumoren und Leberzirrhosen. Eradikation von Coxsackieviren bei Kardiomyopathien, Eradikation von Coxsackieviren bei Diabetes-mellitus-Patienten, Eradikation von Immunschwäche-Viren in Mensch und Tier, Behandlung von Begleitinfektionen in AIDS-Patienten, Behandlung von Entzündungen viraler Genese des Respirationstraktes (Larynxpapillome, Hyperplasien, Rhinitis, Pharyngitis, Bronchitis, Pneumonien), der Sinnesorgane (Keratokonjunktivitis), des Nervensystems (Poliomyelitis, Meningoenzephalitis, Enzephalitis, subakute sklerosierende Panenzephalitis. SSPE, progressive multifokale Leukoenzephalopathie, Lymphozytäre Choriomeningitis), des Magen-Darm-Traktes (Stomatitis, Gingivostomatitis, Ösophagitis, Gastritis, Gastroenteritis, Durchfallerkrankungen), der Leber und des Gallensystems (Hepatitis, Cholangitis, hepatozelluläres Karzinom), des lymphatischen Gewebes (Mononukleose, Lymphadenitis), des hämatopoetischen Systems, der Geschlechtsorgane (Mumpsorchitis), der Haut (Warzen, Dermatitis, Herpes labialis, Fieberbläschen, Herpes Zoster, Gürtelrose), der Schleimhäute (Papillome, Konjunktivapapillome, Hyperplasien, Dysplasien), des Herz-Blutgefäß-Systems (Arteriitis, Myokarditis, Endokarditis, Perikarditis), des Nieren-Harnweg-Systems, der Geschlechtsorgane (Anogenitale Läsionen, Warzen, Genitalwarzen, spitzen Kondylome, Dysplasien, Papillome, Zervixdysplasien, Condylomata acuminata, Epidermodysplasia verruciformis), der Bewegungsorgane (Myositis, Myalgien), Behandlung der Maul- und Klauenseuche der Paarhufer, des Colorado-Zeckenfiebers, des Dengue-Syndroms, des hämorrhagisches Fiebers, der Frühsommermeningoenzephalitis (FSME) und des Gelbfiebers.

Die beschriebenen Verbindungen, d.h. die phosphororganischen Verbindungen nach Formel (I), (III) und (IV) und Ester und Amide derselben an der Phosphinogruppe sowie Salze derselben zeigen eine starke zytotoxische Wirksamkeit gegenüber ein- und mehrzelligen Parasiten, insbesondere gegenüber den Erregern der Malaria und der Schlafkrankheit. Demgemäß sind die erfindungsgemäßen Verbindungen für die Behandlung von Infektionskrankheiten brauchbar, die durch Viren, Bakterien, Parasiten und Pilze bei Mensch und Tier verursacht werden. Die Verbindungen sind auch für den Einsatz zur Vorbeugung von Erkrankungen, die durch Viren, Bakterien, Parasiten und Pilze hervorgerufen werden, insbesondere als Malariaprophylaxe und als Schlafkrankheitsprophylaxe geeignet.

Die erfindungsgemäßen phosphororganischen Verbindungen, hierzu gehören im allgemeinen pharmazeutisch verträgliche Salze, Amide, Ester, ein Salz eines solchen Esters, oder aber Verbindungen, die bei Applikation die erfindungsgemäßen Verbindungen als StoffwechselProdukte oder Abbauprodukte bereitstellen, auch "Prodrugs" genannt, können für die Verabreichung in irgendeiner geeigneten Weise analog zu bekannten antiinfektiös wirkenden Mitteln (gemischt mit einem nicht toxischen pharmazeutisch akzeptablen Träger) zubereitet werden.

Zu pharmazeutisch akzeptablen Salzen der Verbindungen gehören Salze, die die erfindungsgemäßen Verbindungen der Formeln (I), (III) und (IV) in ihrer protonierten Form als Ammoniumsalz anorganischer oder organischer Säuren, wie Salzsäure, Schwefelsäure, Zitronensäure, Maleinsäure, Fumarsäure, Weinsäure, p-Toluolsulfonsäure, bilden.

Pharmazeutisch besonders geeignet sind auch die Salze, die durch geeignete Auswahl von X₃ und X₄ gebildet werden, wie Natriumsalz, Kaliumsalz, Calciumsalz, Ammoniumsalz, Ethanolaminsalz, Triethylaminsalz, Dicyclohexylaminsalz und Salze einer Aminosäure wie Argininsalz, Asparaginsäuresalz, Glutaminsäuresalz.

Die Aktivität der Substanzen wird in einem Versuchssystem bestimmt. Dieses System beruht auf die Messung der Inhibition des Wachstums von Bakterien, Parasiten, Viren, Pilze oder Pflanzen in vitro. Hierzu werden zum Teil Versuchsverfahren verwendet, die dem Fachmann bekannt sind.

Zum Beispiel wird zur Bestimmung der Antimalaria-Aktivität die Inhibition des Wachstums von Malaria-Parasiten in Blutkulturen bestimmt.

Die Bestimmung der antibakteriellen Aktivität beruht auf Messung der Hemmung von Bakterienwachstum auf Nährböden und in Flüssigkulturen.

Die Bestimmung der antiviralen Aktivität beruht auf Inhibition der Bildung von viralen Elementen in Zellkulturen.

Die Bestimmung der fungiziden Aktivität beruht auf Inhibition des Wachstums von Pilzen auf Nährböden und in Flüssigkulturen.

Einige der Mikroorganismen, die untersucht werden sollen, können nur in Tiermodellen untersucht werden. Hier werden die entsprechenden Modelle angewendet.

Substanzen, die eine Wirksamkeit in den in vitro Meßsystemen zeigen, werden in in vivo Modellen weiter untersucht. Die antiparasitäre, antivirale, fungizide oder antibakterielle Aktivität wird in den entsprechenden Tiermodelle weiter evaluiert.

Das Screening nach herbizider Aktivität wird mittels Algensystemen und Messung der Isoprenemission von Pflanzen unter Standardbedingungen bestimmt.

Die pharmazeutisch wirksamen Mittel können in Form von pharmazeutischen Zubereitungen in Dosierungseinheiten zubereitet werden. Dies bedeutet, daß die Zubereitung in Form einzelner Teile, z. B. Tabletten. Dragees. Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füllund Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser. Ethylalkohol, Propylenglykol, Suspendiermittel, z. B. ethoxylierte Isostearylalkohole. Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die Wirkstoffe der Formeln (I), (III) und (IV) sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-%, der Gesamtmischung vorhanden sein.

Die pharmazeutischen Zubereitungen können außer den Verbindungen der Formel (I), (III) und (IV) auch weitere pharmazeutische Wirkstoffe enthalten.

Die Verbindungen können mit bisher beschriebenen Substanzen mit antibakterieller, antiviraler, antimyktoischer und antiparasitärer Eigenschaften verwendet werden. Hierzu gehören insbesondere Verbindungen, die bereits in der Therapie Anwendung gefunden haben oder noch angewendet werden. Hierzu sind insbesondere geeignet Stoffe, die in der in der Roten Liste oder in Simon/Stille, Antibiotika-Therapie in Klinik und Praxis, 9.Auflage 1998 Schattauer Verlag, oder unter http:/www.customs.treas.gov/imp-exp/rulings/harmoniz/hrm129.html im Internet mitaufgeführt. Insbesondere können die Derivate mit Penicilline, Benzylpenicillin (Penicillin G), Phenoxypenicilline, Isoxazolylpenicilline, Aminopenicilline, Ampicillin, Amoxixillin, Bacampicillin, Carboxypenicillin, Ticarcillin, Temocillin, Acyalaminopenicilline, Azlocillin, Mezlocillin, Piperacillin, Apalcillin, Mecillinam, Cephalosporine, Cefazolin-Gruppe, Cefuroxim-Gruppe, Cefoxitin-Gruppe, Cefoxitin, Cefotetan, Cefmetazol, Latamoxef, Flomoxef, Cefotaxim-Guppe, Cefozidim, Ceftazidim-Gruppe, Ceftazidim, Cefpirom, Cefepim, übrige Cephalosporine, Cefsulodin, Cefoperazon, Oralcephalosporine der Cefalexin-Gruppe, Loracarbef, Cefprozil, neue Oralcephalosporine mit erweitertem Spektrum, Cefixim, Cefpodoxim-Proxetil, Cefuroxim-Axetil, Cefetamet, Cefotiam-Hexetil, Cefdinir, Ceftibuten, andere β-Lactam-Antibiotika, Carbapenem, Imipenem /Cilastatin, Meropenem, Biapenem, Aztreonam, β-Lactamase-Hemmer, Clavulansäure/Amoxicillin, Clavulansäure/Ticarcillin, Sulbactam/Ampicillin, Tazobactam/Piperacillin, Tetracycline, Oxytetracyclin, Rolitetraxyxlin, Doxycyclin, Minocyclin, Chloramphenicol, Aminoglykoside, Gentamicin, Tobramycin, Netilmicin, Amikacin, Spectinomyxin, Makrolide, Erythromycin, Clarithromycin, Roxithromycin, Azithromycin, Dirithromycin, Spiramycin, Josamycin, Lincosamide, Clindamycin, Fusidinsäure, Glykopeptid-Antibiotika, Vancomycin, Tecoplanin, Pristinamycin-Derivate, Fosfomycin, Antimikrobielle Folsäureantagonisten, Sulfonamide, Co-Trimoxazol, Trimethoprim, andere Diaminopyrimidin-Sulfonamid-Kombinationen, Nitrofurane, Nitrofurantoin, Nitrofurazon, Gyrase-Hemmer (Chinolone), Norfloxacin, Ciprofloxacin, Ofloxacin, Sparfloxacin, Enoxacin, Fleroxacin, Pefloxacin, Lomefloxacin, Bay Y3118, Nitroimidazole, antimykobakterielle Mittel, Isoniazid, Rifampicin, Rifabutin, Ethambutol, Pyrazinamid, Streptomycin, Capreomycin, Prothionamid, Terizidon, Dapson, Clofazimin, Lokalantibiotika, Bacitracin, Tyrothricin, Polymyxine, Neomycin, Kanamycin, Paromomycin, Mupirocin, antivirale Mittel. Acyclovir, Ganciclovir, Azidothymidin, Didanosin, Zalcitabin, Thiacytidin, Stavudin, Ribavirin, Idoxuridin, Trifluridin, Foscarnet, Amantadin, Interferone, Tibol-Derivate, Proteinase-Inhibitoren, Antimykotika, Polyene, Amphothericin B, Nystatin, Natamycin, Azole, Azole zur septischen Therapie, Miconazol, Ketoconazol, Itraconazol, Fluconazol, UK-109,496, Azole für lokale Anwendung, Clotrimazol, Econazol, Isoconazol, Oxiconazol. Bifonazol, Flucytosin, Griseofulvin, Ciclopiroxolamin, Tolnaftat, Naftifm, Terbinafin, Amorolfin, Antrachinone, Betulinic acid, Semianthrachinone, Xanthone, Naphtoquinone, Aryaminoalkohole, Chinin, Quinidine, Mefloquin, Halofantrin, Chloroquin, Amodiaquin, Acridin, Benzonaphthyridin, Mepacrin, Pyronaridin, Dapson, Sulfonamide, Sulfadoxin, Sulfalene, Trimethoprim, Proguanil, Chlorproguanil, Diaminopyrimidine, Pyrimethamin, Primaquin, Aminoquinoline, WR 238,605, Tetracyclin, Doxycyclin, Clindamycin, Norfloxacin, Ciprofloxacin, Ofloxacin, Artemisinin, Dihydroartemisinin, 10b artemether, Arteether, Atrtesunat, Atovaquon, Suramin, Melarsoprol, Nifurtmox, Stibogluconat-Natrium, Pentamidin, Amphotericin B, Metronidazol. clioquinol, Mebendazol, Niclosamid, Praziquantel, Pyrantel, Tiabendazol, Diethylcarbamazin. Ivermectin, Bithionol, Oxamniquin, Metrifonat. Piperazin, Embonat.

Ferner können die phosphororganischen Verbindungen in den pharmazeutischen Mitteln in Kombination mit Sulfonamid, Sulfadoxin, Artemisinin, Atovaquon, Chinin, Chloroquin, Hydroxychloroquin, Mefloquin, Halofantrin, Pyrimethamin, Armesin, Tetracycline, Doxycyclin, Proguanil, Metronidazol, Praziquantil, Niclosamid, Mebendazol, Pyrantel, Tiabendazöl, Diethylcarbazin, Piperazin, Pyrivinum, Metrifonat, Oxamniquin, Bithionol oder Suramin oder mehreren dieser Substanzen vorliegen.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen. Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate. Granulate. Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I), (III) und (IV) in Gesamtmengen von etwa 0.05 bis etwa 600, vorzugsweise 0,5 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 200, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Patienten, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Die erfindungsgemäßen Verbindungen können in den üblichen Konzentrationen und Zubereitungen bei Tieren zusammen mit dem Futter bzw. mit Futterzubereitungen oder mit dem Trinkwasser gegeben werden.

Ferner können die erfindungsgemäßen Verbindungen hervorragend als Bakterizide, Fungizide und Herbizide bei Pflanzen eingesetzt werden.

Bei Kenntnis der Verbindungsstruktur ist es dem Fachmann allgemein möglich, ein Herstellungsverfahren analog zu bekannten Verfahren zu entwickeln. Im folgenden wird beispielhaft die Herstellung einiger erfindungsgemäßer Verbindungen angegeben:

### Beispiel 1: 5-[2-(Phosphono)ethyl]-N-hydroxy-pyrrolidin-2-on (1)

### N-Fluorenylmethoxycarbonyl-pyrrolidin-2-yl-methanol (1a)

Zu einer Lösung von 182 g (1.8 mol) Pyrrolidin-2-yl-methanol in 1200 ml Dioxan und 1800 ml einer 10%igen Natriumcarbonat-Lsg. tropft man langsam unter Eiskühlung eine Lösung von 476 g (1.85 mol) Chlorameisensäure-1-(9-Fluorenylmethyl)-ester (F-MOC) in 1 l Dioxan. Man läßt 4 h bei dieser Temperatur und 8 h bei Raumtemperatur rühren, gießt auf 1.5 l Eiswasser und extrahiert mit Diethylether. Die eisgekühlte wäßrige Phase wird mit verd. HCl schwach angesäuert, bei 0 °C über Nacht stehengelassen und dann das Produkt **1a** in guter Reinheit und Ausbeute abfiltriert.

### O-(Methansulfonylmethyl)-N-Fluorenylmethoxycarbonyl-pyrrolidin (1b)

470g (1.4 mol) **1a** werden in 300 ml absolutem Pyridin aufgenommen und in der Kälte mit 400 g (3.5 mol) Methansulfochlorid versetzt. Das Gemisch wird unter Argon zunächst 16 h bei 0 °C dann weitere 3 h bei RT gerührt. Nachdem man die Mischung auf Eis gegossen hat, wird mehrmals mit Diethylether extrahiert und die organische Phase nacheinander mit eiskalter verdünnter HCl, NaHCO₃ und Wasser gewaschen. Nach Trocknen über MgSO₄ und Einengen erhält man **1b,** das durch Umkristallisieren aus Aceton/Petrolether gereinigt werden kann.

### 2-(Iodmethyl)-N-Fluorenylmethoxycarbonyl-pyrrolidin (1c)

Zu einer Lösung von 331 g (0.8 mol) **1b** in Aceton tropft man 3 Äquivalente (359.7 g) NaI in Aceton. Nach 12stündigem Rühren bei RT wird filtriert, das Filtrat in 800 ml Wasser gegeben, das mit Natriumthiosulfat versetzt war. Nach mehrmaligem Ausschütteln mit Diethylether werden diese vereinigt, mit Wasser gewaschen, über MgSO₄ getrocknet und unter reduziertem Druck eingeengt. Stehenlassen bei tiefen Temperaturen ergibt zunächst ein Öl, das aus Petrolether umkristallisiert werden kann.

### 2-[2-(Dimethylphosphono)ethyl]-pyrrolidin (1d)

Zu 37.2 g (0.3 mol) Methanphosphonsäuredimethylester in 900 ml trockenem THF werden unter Argon bei -78 °C 190 ml einer 1.6 M Lösung von n-Butyllithium in Hexan (entspricht 0,30 mol) zugetropft. Zur vollständigen Bildung des Carbanions wird 15 min bei gleicher Temperatur weitergerührt.
Zu dieser Lösung werden bei -78°C unter Rühren 133.9 g (0.3 mol) **1c** in 300 ml trockenem THF zugetropft. Nach Aufwärmen auf Raumtemperatur wird 4 h weitergerührt. Anschließend gibt man 85.15 g (1 mol) Piperidin hinzu und läßt über Nacht rühren. Es wird filtriert, das Filtrat wird auf 2 l Wasser gegossen, man trennt die organische Phase ab und extrahiert die wäßrige Phase 4mal mit je 100 ml Dichlormethan. Nach dem Trocknen der vereinigten organischen Phasen über MgSO₄ wird das Lösungsmittel entfernt und der Rückstand im Vakuum fraktioniert destilliert. 2-[2-(Dimethylphosphono)ethyl]-pyrrolidin (**1d**) wird als farbloses Öl in Ausbeuten von 30-40 % erhalten.

### 5-[2-(Dimethylphosphono)ethyl]pyrrolidinon (1e)

Zu einer auf 0 °C gekühlten Lösung von 3.32 g (15 mmol) **1d** in 50 ml trockenem Aceton wird eine Lösung von 60 mmol Dimethyldioxiran in 120 ml trockenem Aceton getropft. Man läßt 30 min bei 0°C rühren und zieht dann das Lösungsmittel im Vakuum ab. Das erhaltene Rohprodukt wird aus Isopropanol umkristallisiert. Man erhält 5-[2-(Dimethylphosphono)ethyl]pyrrolidinon (**1e**) in mäßiger Ausbeute als farblose Kristalle. Dimethyldioxiran wird nach einer Vorschrift nach Org.Syntheses IX, 288 hergestellt.

### 5-[2-(Phosphono)ethyl]-N-hydroxy-pyrrolidin-2-on (1f)

Zu einer auf 0 °C gekühlten Lösung von 1.19 g (5 mmol) **1e** in 50 ml trockenem Acetonitril tropft man 3.06 g (20 mmol) Trimethylbromsilan. Man läßt 3 h bei RT rühren, zieht dann das Lösungsmittel im Vakuum ab, nimmt mit 20 ml Eiswasser auf, läßt 1 h bei Raumtemperatur rühren, ethert zweimal mit je 20 ml Ether aus. stellt mit 2 M NaOH einen pH-Wert von 4.5 ein und zieht dann am Rotavapor bei maximal 50°C das Wasser im Vakuum ab. Der feste Rückstand wird aus Methanol/Essigester kristallisiert. Man erhält 5-[2-(Phosphono)ethyl]-N-hydroxy-pyrrolidin-2-on (**1f**) in Form gelblicher Mikrokristalle in guter Ausbeute.

### Beispiel 2: 3-(Phosphonomethyl)-N-hydroxy-pyrrolidin-2-on (2)

### 3-Methyl-N-(2-trimethylsilylethoxy)-pyrrolidin-2-on (2a)

Zu einer Lösung von 20.37 g (120 mmol) O-(2-Trimethylsilyethyl)hydroxylaminhydrochlorid in 100 ml absolutem Ethanol wird unter Feuchtigkeitsausschluß bei 0 °C eine Lösung von 120 mmol Natriumethanolat in 50 ml absolutem Ethanol getropft. Von ausgefallenem NaCl wird über eine Argonfritte filtriert. Das Filtrat wird unter reduziertem Druck von Ethanol befreit und nach dem Belüften mit Argon mit abs. Toluol aufgenommen. Dazu gibt man bei 0°C 1 mol% RhCl₃*3 H₂O, 5 mol% DMAP sowie tropfenweise 5.01 g (50 mmol) 2-Methylbutyrolacton. Man läßt auftauen und rührt über Nacht unter Rückfluß am Wasserabscheider. Nach dem Abkühlen zieht man flüchtige Bestandteile am Rotavapor im Vakuum bei 50°C ab, wobei ein schwach gefärbtes Öl zurück bleibt. Aufnehmen in 50 ml Ether, filtrieren durch eine kurze SiO₂-Säule und Abziehen des Lösungsmittels führt in mäßiger Ausbeute zu 3-Methyl-N-(2-trimethylsilylethoxy)-pyrrolidin-2-on (**2a**) als nahezu farbloses Öl, das in guter Reinheit anfällt.

### 3-Brommethyl-N-(2-trimethylsilylethoxy)-pyrrolidin-2-on (2b)

50 mmol **2a -** gelöst in 30 ml absolutiertem Tetrachlorkohlenstoff - werden mit 1.2 Äquivalenten N-Bromsuccinimid versetzt und 12 h unter Rückfluß erhitzt. Azobisisobutyronitril (AIBN) wird stündlich in kleinen Mengen zugegeben. Nach dem Abkühlen wird von Succinimid filtriert, dieses mit CCl₄ gewaschen und die vereinigten CCl₄-Phasen unter reduziertem Druck eingeengt. Das entstandene Öl kann an SiO₂ chromatographiert werden, wobei man **2b** in schlechter Ausbeute erhält.

### 3-(Diethylphosphonomethyl)-N-(2-trimethylsilylethoxy)-pyrrolidin-2-on (2c)

100 mmol (17.3 ml) Triethylphosphit werden mit 100 mmol **2b** versetzt und ohne Lösungsmittel für 0.5 h auf 150 °C erwärmt. Nach dem Abkühlen wird unter reduziertem Druck eingeengt und das gelblich braune Öl an SiO₂ mit Chloroform/Methanol im Verhältnis 25:1 chromatographiert. Nach Abziehen flüchtiger Bestandteile erhält man in mäßiger Ausbeute **2c** als gelbes Öl.

### 3-(Phosphonomethyl)-N-(2-trimethylsilylethoxy)-pyrrolidin-2-on (2d)

Zu 30 mmol **2c** in 50 ml absolutiertem Acetonitril tropft man unter Eiskühlung 4 Äquivalente (120 mmol. 15.4 ml) Trimethylbromsilan, rührt 15 min bei gleicher Temperatur, dann 2 h bei RT, engt unter reduziertem Druck bis zu einem gelblichen Öl ein, nimmt in 100 ml Wasser auf und hydrolysiert 1 h bei RT (pH kleiner 1). Diese Lsg. wird zweimal mit CHCl₃ extrahiert, einmal mit Wasser zurück extrahiert und die vereinigten wäßrigen Phasen unter reduziertem Druck bei 45 °C eingeengt. Das entstehende gelb-braune Öl wird in Wasser aufgenommen und ein pH von 4.5 bis 5.0 eingestellt. **2d** fällt nach Waschen mit Eiswasser als nahezu farbloses Natriumsalz in 50 %iger Ausbeute an.

### 3-(Phosphonomethyl)-N-hydroxy-pyrrolidin-2-on (2e)

5.3 mmol BF₃-Etherat werden zu einer Lösung von 2.65 mmol **2d** in absolutiertem Acetonitril gegeben und 0.5 h bei Raumtemperatur gerührt. Nach Einengen der Lösung unter reduziertem Druck nimmt man in 40 ml Essigsäureethylester auf, wäscht mit 3%iger Kochsalz-Lsg., trocknet über MgSO₄ filtriert über einen Membranfilter und zieht das Lösungsmittel unter reduziertem Druck ab. Das Rohprodukt läßt sich aus MeOH/EtOH umkristallisieren, wobei man das Produkt in reiner Form und guter Ausbeute erhält.

### Beispiel 3:4-(Phosphonomethyl)-N-hydroxy-pyrrolidin-2-on (3)

### 4-Methyl-2[5H]-furanon (3a)

10 g 3-Methylglutarsäuremonoethylester und 31 g Bleitetraacetat gelöst in 400 ml absolutem CCl₄ werden unter Argon zum sieden erhitzt. Nach 10minütiger Belichtung mittels Wolfram-Tageslicht-Lampe gibt man innerhalb von 45 min 23.1 g Iod unter fortwährender Belichtung zu. Nach dem Abkühlen wird filtriert, das Filtrat mit wäßriger Natriumthiosulfat-Lsg., Soda-Lsg. und Wasser gewaschen, über MgSO₄ getrocknet und unter reduziertem Druck eingeengt. Man erhält gamma-Iod-Ester **3a'** als Öl, das ohne weitere Reinigung weiter umgesetzt werden kann. 18.4 g frisch gefälltes Silberacetat und 24 g Acetanhydrid werden in 82 ml Eisessig für eine Stunde auf 120 °C erwärmt, 19.1 g des gamma-Iod-Esters **3a**' hinzugefügt und 2 weitere Stunden unter Rückfluß erwärmt. Dann wird 15 h bei RT aufbewahrt, mit 300 ml Ether versetzt und filtriert. Nach dem Waschen des Filtrats mit Wasser und wäßriger Soda-Lsg. extrahiert man die wäßrige Phase nochmals mit Ether, trocknet die vereinigten organischen Extrakte über MgSO₄ und engt unter reduziertem Druck ein. Nach Aufnehmen dieses Öls in 70 ml 2 N NaOH, 10 ml EtOH und 50 ml Wasser wird mit Ether mehrmals extrahiert, wobei man die Ether-Phasen quantitativ verwirft. Die mit 150 ml 6 N HCl angesäuerte wäßrige Phase wird mit Ether perforiert und nach Trocknen über MgSO₄ und Einengen unter reduziertem Druck destilliert (102-105 °C bei 34 Torr). Man erhält 4.4 g 4-Methyl-2[5H]-furanon (**3a**).

Die weiteren Schritte zur Darstellung von **3** folgen der unter **2** beschriebenen Synthesesequenz über die Einführung von O-(2-Trimethylsilyethyl)-hydroxylamin-hydrochlorid, NBS, Triethylphosphit und die Hydrolysen von Phosphonsäureester per Trimethylbromsilan und die Freisetzung der cyclischen Hydroxamsäure per BF₃-Etherat. Zur Synthesesequenz vgl. auch: T.Sakamoto, Y.Kikugawa *J. Org. Chem.* **1994,** *59*, 929-931.

### Beispiel 4: N-Hydroxy-3-amino-4-(Phosphonomethyl)-pyrrolidin-2-on (4)

### 2-Phenyl-4-(2-acetoxy-1-acetoxymethyl-ethyliden)-2-oxazolin-5-on (4a)

0.2 mol Hippursäure, 0.6 mol Acetanhydrid. 0.24 mol 1,3-Diacetoxyaceton und 0.1 mol wasserfreies Blei(II)acetat legt man in 500 ml THF vor und erhitzt unter Argon 16 h unter Rückfluß. Nach dem Abkühlen auf RT filtriert man von anorganischen Salzen, engt unter reduziertem Druck ein, nimmt in 500 ml Toluol auf, leitet Schwefelwasserstoffgas ein. bis kein weiteres PbS mehr ausfällt und engt nach dem Filtrieren erneut ein. Das Produkt wird an SiO₂ mit n-Hexan/Chloroform als Lösungsmittelgemisch chromatographiert, wobei **4b** in 73% Ausbeute erhalten wird.
Diacetoxyaceton wird nach einer Vorschrift von A.O.L.Fischer, H.Mildbrand *Ber.Dt.chem.Ges. 57*, 707, **1924** synthetisiert.

### 2-Amino-3-methoxy-buttersäure (4b)

Eine Lösung von 31.8 mol **4a** in 150 ml Dioxan wird mit 1g Pd/C versetzt und so lange unter Normaldruck hydriert, bis 10 mol Wasserstoff aufgenommen worden sind (4-6 h). Nach Filtrieren vom Katalysator wird zur Trockene eingeengt, in 40 ml Wasser und 60 ml konz. HCl aufgenommen, 4 h unter Rückfluß gekocht und über Nacht im Kühlschrank aufbewahrt. Die filtrierte Lösung engt man ein, nimmt in 50 ml Wasser auf und reinigt an Ionentauscher Amberlite IR 120, H⁺ durch Eluieren mit 300 ml wäßriger Ammoniak-Lsg. Man kocht so lange, bis kein Ammoniak mehr nachgewiesen werden kann, engt erneut unter reduziertem Druck ein und kristallisiert um.

### Alpha-Amino-beta-methoxy-gamma-butyrolacton (4c)

25 mmol **4b** rührt man 15 min bei RT mit 20 ml 2.5%iger HCI. Die Lösung wird bis zur Trockene eingeengt und über Nacht per Soxlett mit Chloroform extrahiert. Nach Abziehen des Lösungsmittels unter reduziertem Druck erhält man das Lacton **4c** in nahezu quantitativer Ausbeute.

### N,N-Dibenzylamino-beta-methoxy-gamma-butyrolacton (4d)

0.2 mol **4c.** 72 g K₂CO₃ und 300 mg Tetrabutylammonumiodid sowie 500 mg 18-Krone-6 werden in 100 ml Ethanol suspendiert und auf 40 °C erhitzt. Man tropft innerhalb von 15 min 0.65 mol Benzylbromid zu, rührt 12 h bei RT, trennt die Phasen, wäscht die wäßrige zweimal mit je 75 ml Ether, vereinigt die organischen Phasen, wäscht diese mit gesättigter NaCl-Lsg. und trocknet über MgSO₄. Nach dem Einengen unter reduziertem Druck chromatographiert man an einer kurzen Kieselgel-Säule.

### N,N-Dibenzylamino-beta-(brommethyl)-butyrolacton (4e)

2.23 g (6.18 mmol) CBr₄ wird zu einer Mischung aus 4.12 mmol **4d,** 6.18 mmol PPh₃ und 20 ml abs. Acetonitril gegeben. Man rührt 20 h bei RT, entfernt das Lösungsmittel unter reduziertem Druck und chromatographiert an Silicagel mit Essigester/n-Hexan als Lösungsmittel. **4e** entsteht in schwankender Ausbeute als gelbliches Öl.

### N,N-Dibenzylamino-beta-(dimethylphosphonomethyl)-butyrolacton (4f)

40 mmol **4e** wird mit einem Äquivalent Trimethylphosphit in Toluol 0.5 bis 1 h unter Rückfluß erhitzt. Nach dem Abkühlen wird unter reduziertem Druck eingeengt und das zurückbleibende Öl an SiO₂ mit Chloroform/Methanol als Laufmittel chromatographiert. Nach Abziehen flüchtiger Bestandteile erhält man **4f** in mäßiger Ausbeute.

### N,N-Dibenzylamino-beta-(dimethylphosphonomethyl)-butyrolactam (4g)

Zu einer Lösung von 120 mmol O-Benzylhydroxylamin Hydrochlorid in 100 ml absolutem Ethanol wird unter Feuchtigkeitsausschluß bei 0 °C eine Lösung von 100 ml Natriumethanolat in 50 ml absolutem Ethanol getropft. Von ausgefallenem NaCl wird über eine Argonfritte filtriert. Das Filtrat wird unter reduziertem Druck von Ethanol befreit und nach dem Belüften mit Argon mit abs. Toluol aufgenommen. Dazu gibt man bei 0°C 1 mol% RhCl₃ 3 H₂O, 5 mol% DMAP sowie tropfenweise 50 mmol **4f.** Man läßt auftauen und rührt über Nacht unter Rückfluß am Wasserabscheider. Nach dem Abkühlen zieht man flüchtige Bestandteile am Rotavapor im Vakuum bei 50°C ab, wobei ein Öl zurück bleibt. Aufnehmen in 40 ml Essigester, filtrieren durch eine kurze SiO₂-Säule und Abziehen des Lösungsmittels führt in mäßiger Ausbeute zu **4g** als gelbes Öl, das in guter Reinheit anfällt.

### N-Hydroxy-3-amino-4-(dimethylphosphonomethyl)-pyrrolidin-2-on (4h)

30 mmol **4g** in 60 ml Methanol und 10 ml Ameisensäure werden mit 5 mol% Pd/C (10-20%) unter Normaldruck bei RT 13 h hydriert. Nach Abfiltrieren vom Katalysator engt man unter reduziertem Druck ein und setzt ohne Reinigung weiter um.

### N-Hydroxy-3-amino-4-(Phosphonomethyl)-pyrrolidin-2-on (4i)

Zu 10 mmol **4h** in 20 ml absolutiertem Acetonitril tropft man unter Eiskühlung 35 mmol Trimethylbromsilan, rührt 15 min bei gleicher Temperatur, dann 2 h bei RT, engt unter reduziertem Druck bis zu einem gelblichen Öl ein, nimmt in 100 ml Wasser auf und hydrolysiert 1 h bei RT. Diese Lsg. wird zweimal mit CHCl₃ extrahiert, einmal mit Wasser zurück extrahiert und die vereinigten wäßrigen Phasen unter reduziertem Druck bei 45 °C eingeengt. Das entstehende dunkle Öl wird in Wasser aufgenommen und ein pH von 6.0 eingestellt. Ausfallendes **4i** wird filtriert und mit Eiswasser gewaschen. Man erhält **4i** als Natriumsalz in Form beiger Kristalle in einer Ausbeute von 35-40 %.

### Beispiel 5: N,2-Dihydroxy-5-(2-phosphonoethyl)-pyrrol (5)

### N-Benzyloxy-5-[2-(dimethylphosphono)ethyl]pyrrolidin-2-on (5a)

20 mmol 5-[2-(dimethylphosphono)ethyl]pyrrolidinon (**1e**) werden mit 1.2 Äquivalenten Benzylbromid, 10 mg Tetrabutylammoniumiodid und 1.3 Äquivalenten Triethylamin in 30 ml THF über Nacht bei Raumtemperatur gerührt, dann in Eiswasser gegeben, mehrmals mit kleinen Mengen Ether extrahiert, die Diethylether-Phase mit kalter verd. HCl und mit gesättigter Kochsalz-Lsg. gewaschen, über MgSO₄ getrocknet, eingeengt und das Rohprodukt an Kieselgel mit Chloroform/Methanol 25:1 chromatographiert. Es entsteht in guter Ausbeute **5a.**

### N-Benzyloxy-3-phenylseleno-5-[2-(dimethylphosphono)ethyl]pyrrolidin-2-on (5b)

Zu 2.4 mmol Diisopropylamid in 3 ml THF (LDA, hergestellt aus 0.35 ml Diisopropylamin und 1.6 ml 1.65 M n-BuLi in Hexan unter Argon bei -78 °C) gibt man bei -78 °C 2.0 mmol **5a,** das man in 1 ml absolutem THF löst und rührt 20 min bei gleicher Temperatur. Zum Enolat von **5a** gibt man bei -78 °C 2.4 mmol Diphenyldiselenid, gelöst in 1 ml THF und 1.2 mmol HMPT. Die Reaktionsmischung wird 40 min bei -78 °C und 1.5 h bei -40 °C gerührt. Quenchen mit 0.1 N HCl mit nachfolgendem mehrmaligem Ausethern ergibt nach Chromatographie an Kieselgel ein gelbliches charakteristisch riechendes Öl **5b.**

### N-Benzyloxy-2-hydroxy-4-[2-(dimethylphosphono)ethyl]pyrrol (5c)

0.2 mmol Phenylselenoverbindung **5b -** gelöst in 1 ml absolutem THF - versetzt man mit 30 µl Eisessig, tropft unter Eiskühlung 140 µl Perhydrol (30%ige Wasserstoffperoxid-Lsg.) zu und rührt 30 min bei gleicher Temperatur. Die Lösung gibt man in kalte gesättigte wäßrige Natriumhydrogencarbonat-Lsg., extrahiert mit Ether, trocknet über MgSO₄, engt unter reduziertem Druck ein und chromatographiert das Rohprodukt an Kieselgel. **5c** entsteht in guter Ausbeute als gelbliches Öl.

### N,2-Dihydroxy-4-[2-(dimethylphosphono)ethyl]pyrrol (5d)

0.15 mmol **5c** gibt man in 50 ml absolutes EtOH, fügt eine Spatelspitze 10%iges Pd auf Aktivkohle hinzu und hydriert an einer Normaldruckhydrieranlage bei RT 1 h unter kräftigem Rühren. Nach dem Filtrieren vom Katalysator wird eingeengt und das Rohprodukt ohne weitere Reinigung umgesetzt.

### N,2-Dihydroxy-5-(2-phosphonoethyl)-pyrrol (5e)

Zu 15 mmol **5d** in 50 ml absolutiertem Acetonitril tropft man unter Eiskühlung 4 Äquivalente (60 mmol, 8 ml) Trimethylbromsilan, rührt 15 min bei gleicher Temperatur, dann 2 h bei RT, engt unter reduziertem Druck bis zu einem gelblichen Öl ein, nimmt in 80 ml Wasser auf und hydrolysiert 1 h bei RT (pH < 1). Diese Lsg. wird zweimal mit CHCl₃ extrahiert, einmal mit Wasser zurück extrahiert und die vereinigten wäßrigen Phasen unter reduziertem Druck bei max. 45 °C eingeengt. Das entstehende Öl wird in Wasser aufgenommen und mit NaHCO₃ ein pH von 4.5 bis 5.0 eingestellt. **5e** wird abgesaugt und fällt nach Waschen mit Eiswasser als nahezu farbloses Natriumsalz **5e** in einer Ausbeute von 40 % an.

### Beispiel 6: N-Hydroxy-3-[2-(phosphono)ethyl]-1H-pyridon (6)

### 2-Brom-3-(brommethyl)pyridin (6a)

10 g (58.1 mmol) 2-Brom-3-methylpyridin und 11.4 g (64 mmol) werden in 250 ml CCl₄ 24 h unter Rückfluß erhitzt. Von Succinimid wird filtriert und die organische Phase zweimal mit Wasser gewaschen. Nach dem Einengen unter reduziertem Druck wird **6a** durch fraktionierte Destillation als farblose Flüssigkeit erhalten (Sdp.: 90 °C, 1 Torr).

### 2-Brom-3-[2-(dimethylphosphono)ethyl]pyridin (6b)

Zu 100 ml absolutem THF tropft man bei -20 °C 0.21 mol MeLi in Ether, gibt 0.1 mol Trimethylphosphit, gelöst in 50 ml THF so innerhalb von 15 min zu, daß die Innentemperatur langsam 0 °C erreicht. Dann tropft man bei einer Temperatur von -78 °C 0.107 mol **6a** in 20 ml THF zu, rührt weitere 30 min bei gleicher Temperatur, läßt auftauen und quencht bei 0 °C durch tropfenweise Zugabe von 80 ml 3 M HCI. Die organische Phase wird abgetrennt, die wäßrige dreimal mit je 40 ml Dichlormethan extrahiert und die vereinten organischen Phasen nach Trocknen über MgSO₄ eingeengt. Das gelbe Rohprodukt kann an einer kurzen Säule an SiO₂ gereinigt werden.

### 2-Brom-3-[2-(dimethylphosphono)ethyl]pyridin-N-oxid (6c)

100 mmol **6b** in 60 ml Eisessig werden mit 2 Äquivalenten einer 40%igen Peressigsäure-Lsg. versetzt, wobei eine Temperatur von 50 °C nicht überschritten werden sollte. Nach 5stündigem Erwärmen auf 50 °C und 12stündigem Erwärmen auf 70 °C wird die Lösung unter reduziertem Druck auf die Hälfte eingeengt, auf Eis gegeben und mit 40%iger wäßriger KOH stark alkalisch gemacht. Dreimalige Extraktion mit Chloroform ergibt nach Trocknen über K₂CO₃ und dem Einengen unter reduziertem Druck ein Öl von N-Oxid **6c,** das ohne weitere Reinigung umgesetzt wurde.

### N-Hydroxy-3-[2-(dimethylphosphono)ethyl]-1H-pyridon (6d)

In absolutem MeOH erwärmt man im Glasautoklaven **6c** zusammen mit gemörsertem Kaliumhydroxid. Kaliumcarbonat sowie Tris(3,6-dioxaheptyl)amin 3 h auf 120 °C. Nach dem Abkühlen gibt man die Reaktionsmischung auf Wasser, stellt einen pH-Wert von 6 ein, engt im Vakuum ein und erhält nach Zugabe von Ethanol ein Rohprodukt, das sich aus Ethanol/Toluol in mäßiger Ausbeute umkristallisieren läßt.

### N-Hydroxy-3-[2-(phosphono)ethyl]-1H-pyridon (6e)

Zu 10 mmol **6d** in 30 ml absolutem Acetonitril tropft man unter Eiskühlung 40 mmol Trimethylbromsilan. rührt 15 min bei gleicher Temperatur, dann 2 h bei RT, engt unter reduziertem Druck bis zu einem Öl ein, nimmt in 20 ml Wasser auf und hydrolysiert 1 h bei RT (pH sauer). Diese Lsg. wird zweimal mit CHCl₃ extrahiert, einmal mit Wasser zurück extrahiert und die vereinigten wäßrigen Phasen unter reduziertem Druck bei 45 °C eingeengt. Das entstehende braune Öl wird in Wasser aufgenommen zweimal mit Aktivkohle gerührt, davon filtriert und ein pH von 5 eingestellt. **6e** fällt dabei aus, wird filtriert, mit Eiswasser gewaschen und kann aus MeOH/EtOH umkristallisiert werden.

### Beispiel 7: N-Hydroxy-6-[2-(phosphono)ethyl]-1H-pyridon (7)

### 2-Brom-6-brommethylpyridin (7a)

10.1 g (58.7 mmol) 2-Brom-6-Methylpyridin. 11.1 g (62.4 mmol N-Bromsuccinimid (NBS) sowie 0.1 g (0.6 mmol) AIBN werden in 150 ml Toluol 6 h unter Argon auf 110 °C erwärmt, wobei mit einer Wolfram-Tageslichtlampe (150 W, > 320 nm) gleichzeitig belichtet wird. Nach dem Abkühlen filtriert man Succinimid ab und engt die Lösung unter reduziertem Druck ein. Chromatographie an Kieselgel (Laufmittel: Hexan/Dichlormethan ergibt zunächst 2-Brom-6-dibrommethylpyyridin, später läßt sich **7a** in einer Ausbeute von bis zu 45 % eluieren (Smp.: 138 °C).

### 2-Brom-6-[2-(dimethylphosphono)ethyl]pyridin (7b)

Zu 100 ml absolutem THF tropft man bei -20 °C 0.21 mol MeLi in Ether, gibt 0.1 mol Trimethylphosphit, gelöst in 50 ml THF so innerhalb von 15 min zu, daß die Innentemperatur langsam 0 °C erreicht. Dann tropft man bei einer Temperatur von -78 °C 0.107 mol **7a** in 15 ml THF zu, rührt weitere 30 min bei gleicher Temperatur, läßt auftauen und quencht bei 0 °C durch tropfenweise Zugabe von 80 ml 3 M HCl. Die organische Phase wird abgetrennt, die wäßrige mehrmals mit je 40 ml Dichlormethan extrahiert und die vereinigten organischen Phasen nach Trocknen über MgSO₄ eingeengt. Das gelbe Rohprodukt kann an SiO₂ chromatographisch gereinigt werden, wobei **7b** in einer Ausbeute von 47 % entsteht.

### 2-Brom-6-[2-(dimethylphosphono)ethyl]pyridin-N-oxid (7c)

50 mmol **7b** in 50 ml Eisessig werden mit 2 Äquivalenten einer 40%igen Peressigsäure-Lsg. versetzt, wobei sich die Temperatur zw. 25 und 45 °C bewegt. Nach 5stündigem Erwärmen auf 50 °C und 12stündigem Erwärmen auf 70 °C wird die Lösung auf Eis gegeben und mit 40%iger wäßriger KOH stark alkalisch gemacht. Dreimalige Extraktion mit Chloroform ergibt nach Trocknen über K₂CO₃ und Einengen unter reduziertem Druck ein Öl von N-Oxid **7c,** das aus Ether/Ethanol umkristallisiert werden kann.

### N-Hydroxy-6-[2-(dimethylphosphono)ethyl]-1H-pyridon (7d)

In absolutem MeOH erwärmt man **7c** im Glasautoklaven zusammen mit gemörsertem Kaliumhydroxyd. Kaliumcarbonat sowie Tris(3,6-dioxaheptyl)amin 2.5 h auf 100 °C. Nach dem Abkühlen gibt man die Reaktionsmischung in Wasser, stellt einen pH-Wert von 6 ein, engt im Vakuum ein und erhält nach Zugabe von Ethanol ein Rohprodukt, das sich analog zu **6d** in mäßiger Ausbeute umkristallisieren läßt.

### N-Hydroxy-6-[2-(phosphono)ethyl]-1H-pyridon (7e)

Zu 10 mmol **7d** in 25 ml absolutem Acetonitril tropft man unter Eiskühlung 40 mmol Trimethylbromsilan, rührt 10 min bei gleicher Temperatur, dann 2 h bei RT, engt unter reduziertem Druck bei 45 °C bis zu einem Öl ein, nimmt in 20 ml Wasser auf und hydrolysiert 1 h bei RT. Diese Lsg. wird zweimal mit CHCl₃ extrahiert, einmal mit Wasser zurück extrahiert und die vereinigten wäßrigen Phasen unter reduziertem Druck bei 45 °C eingeengt. Das entstehende dunkel gefärbte Öl wird in Wasser aufgenommen und ein pH von 4.8 eingestellt. **7e** fällt dabei als Natriumsalz aus. Nach Filtration und Waschen mit Eiswasser erhält man **7e** als Rohprodukt, das aus MeOH/Toluol umkristallisiert werden kann.

### Beispiel 8: N-Hydroxy-5-[2-phosphono-2-hydroxy)ethyl]pyrrolidin-2-on (8)

### N-Benzyl-2-(1,3-dithioylmethyl)-pyrrolidin (8a)

100 mmol (12,0 g) 1,3-Dithian werden unter Schutzgas abgewogen, 250 ml absolutes THF dazu gegeben und bei -40 °C ein 5%iger Überschuß von n-BuLi in Hexan innerhalb von 3-5 min hinzugetropft. Man läßt 2 h bei -25 bis -15 °C rühren, kühlt auf -60 bis -78 °C und gibt 100 mmol 2-Iodmethyl-N-benzyloxy-pyrrolidin langsam unter Schutzgas hinzu. Nach 5-6stündigem Rühren bei -20 bis -10 °C läßt man auf 0 °C erwärmen und stellt die Reaktionsmischung drei Tage in den Kühlschrank. Nach dem Einengen auf ca. 20 ml gibt man diese auf das dreifache Volumen an Wasser, extrahiert drei- bis fünfmal mit Chloroform, vereinigt die organischen Phasen, wäscht nacheinander mit Wasser, 6%iger KOH sowie erneut mit Wasser und trocknet die Chloroform-Phase über K₂CO₃. Der nach dem Einengen unter reduziertem Druck erhaltene Rückstand wird ohne weitere Reinigung umgesetzt.

### N-Benzyl-2-(formylmethyl)-pyrrolidin (8b)

Zu einer Lösung von 9 mmol **8a** in 30 ml THF und 6 ml Wasser gibt man bei RT 1.1 g CaCO₃ und 2.5 ml Hg(ClO₄)₂ einer 4 M wäßrigen Lsg., rührt weitere 5 bis 10 min, gibt 150 ml Ether hinzu und filtriert von anorganischen Salzen. Nach Einengen der Lösung unter reduziertem Druck bleibt ein farbiges Rohprodukt zurück, das per Flashchromatographie gereinigt wird.

### N-Benzyl-2-[2-(diethylphosphono)-2-hydroxy]pyrrolidin (8c)

20 g (145 mmol) Diethylphosphonat und 140 mmol **8b** werden 8 h unter Argon auf 80 bis 85 °C erwärmt. Nach dem Abkühlen engt man unter reduziertem Druck ein und reinigt das Produkt **8c** chromatographisch an Kieselgel.

### N-Benzyl-2-[2-(diethylphosphono)-2-acetoxyl]pyrrolidin (8d)

Eine Mischung aus 50 mmol von 1-Hydroxyphoshonsäureester **8c,** 75 mmol Triethylamin, 75 mmol Acetanhydrid und 4 mmol Dimethylaminopyridin (DMAP) wird 14 h bei RT stehen gelassen und nach Zugabe von 100 ml Ether und 2 N HCl wäscht man die etherische Phase mit gesättigter wäßriger NaHCO₃-Lsg., trocknet über MgSO₄, engt ein und reinigt an einer kurzen Alox-Säule.

### 2-[2-(diethylphosphono)-2-acetoxy]pyrrolidin (8e)

Eine Lösung von 40 mmol **8d** in 30 ml Eisessig wird nach Zugabe von 400 mg PtO₂ 6 h unter Normaldruck bei 70 °C hydriert. Nach Filtration von Katalysator wird in stark alkalischem Medium mehrmals ausgeethert, die vereinigten Etherextrakte über MgSO₄ getrocknet, eingeengt und das in guter Ausbeute entstehende Produkt **8e** direkt weiter umgesetzt.

### N-Hydroxy-5-[(2-diethylphosphono-2-acetoxy)-ethyl]pyrrolidin-2-on (8f)

Zu einer auf 0 °C gekühlten Lösung von 20 mmol **8e** in 50 ml trockenem Aceton wird eine Lösung von 70 mmol Dimethyldioxiran in 110 ml trockenem Aceton getropft. Man läßt 30 min bei 0°C rühren und zieht dann das Lösungsmittel im Vakuum ab. Man erhält ein gelbes Öl, das an Kieselgel mit einem Laufmittelgemisch Chloroform/Methanol gereinigt werden kann.

### N-Hydroxy-5-[(2-diethylphosphono-2-hydroxy)-ethyl]pyrrolidin-2-on (8g)

Das gelbe Öl **8f** wird mit 5 M wäßriger KOH in MeOH bei RT über Nacht gerührt, dann neutralisiert, von MeOH unter reduziertem Druck befreit und ausgeethert. Die vereinigten organischen Phasen trocknete man über MgSO₄ und engte bis zur Trockene ein. Entstandenes **8g** wird ohne weitere Reinigung für die folgenden Umsetzungen verwendet.

### N-Hydroxy-5-[(2-phosphono-2-hydroxy)-ethyl]pyrrolidin-2-on (8h)

Zu einer auf 0 °C gekühlten Lösung von 20 mmol **8g** in 50 ml trockenem Acetonitril tropft man 80 mmol Trimethylbromsilan. Man läßt 3 h bei RT rühren, zieht dann das Lösungsmittel im Vakuum ab, nimmt mit 60 ml Eiswasser auf. läßt 1 h bei Raumtemperatur rühren, extrahiert dreimal mit je 60 ml Ether aus, stellt mit 2 M NaOH einen pH-Wert von 5.5 bis 6.0 ein und zieht dann am Rotavapor bei maximal 45 °C das Wasser unter reduziertem Druck ab. Der feste Rückstand wird aus Methanol/Essigester kristallisiert. Man erhält N-Hydroxy-5-[(2-phosphono-2-hydroxy)-ethyl]pyrrolidin-2-on (**8h**) in Form gelblich-weißer Mikrokristalle in guter Ausbeute.

### Beispiel 9: 3-(Methylphosphono)-N-hydroxy-succinimid (9)

### 3-(Brommethyl-Bernsteinsäureanhydrid (9a)

Analog zur Darstellung von 3-Brommethyl-N-(2-trimethylsilylethoxy)-pyrrolidin-2-on (**2b**) setzt man
50 mmol 2-Methylbernsteinsäureanhydrid - gelöst in 30 ml absolutiertem Tetrachlorkohlenstoff - mit 1.2 Äquivalenten N-Bromsuccinimid um, indem man 12 h unter Rückfluß erhitzt. Azobisisobutyronitril (AIBN) wird stündlich in kleinen Mengen zugegeben. Nach dem Abkühlen wird von Succinimid filtriert, dieses mit CCl₄ gewaschen und die vereinigten CCl₄-Phasen unter reduziertem Druck eingeengt. Das entstandene Öl kann an SiO₂ chromatographiert werden, wobei man **9a** in geringer Ausbeute erhält.

### 3-[2-(Dimethylphosphono)ethyl]-bernsteinsäureanhydrid (9b)

40 mmol **9a** wird mit einem Äquivalent Trimethylphosphit in Toluol 0.5 bis 1 h unter Rückfluß erhitzt. Nach dem Abkühlen wird unter reduziertem Druck eingeengt und das gelbliche Öl an SiO₂ chromatographiert. Nach Abziehen flüchtiger Bestandteile erhält man **9b** in geringer Ausbeute.

### 3-[2-(Dimethylphosphono)methyl]-N-benzyloxy-succinimid (9c)

1.0 g Benzyloxyamin wird mit 1.0 Äquivalenten **9b** im Glasautoklaven für 30 min auf 180 °C erwärmt. Nach dem Abkühlen engt man das Öl unter reduziertem Druck ein, wobei man eine geringe Rohprodukt-Ausbeute beobachtet und setzt **9c** ohne Aufreinigung weiter um.

### 3-[2-(Dimethylphosphono)methyl]-N-hydroxy-succinimid (9d)

9.28 mmol der Benzyloxyverbindung **9c** gelöst in 60 ml Ethanol werden mit 700 mg Pd/C versetzt und für 4 h einer Normaldruckhydrierung bei RT unterzogen. Nachdem kein Wasserstoff mehr aufgenommen wird, filtriert man vom Katalysator ab, engt unter reduziertem Druck ein und kristallisiert aus Essigester/Hexan um, wobei man **9d** in guter Ausbeute erhält.

### 3-[2-Phosphonomethyl]-N-hydroxy-succinimid (9e)

Zu einer auf 0 °C gekühlten Lösung von 30 mmol **9d** in 70 ml trockenem Acetonitril tropft man 110 mmol Trimethylbromsilan. Man läßt 3 h bei RT rühren, zieht dann das Lösungsmittel im Vakuum ab, nimmt mit 80 ml Eiswasser auf, läßt 1 h bei Raumtemperatur rühren, extrahiert dreimal mit je 50 ml Ether, stellt mit NaHCO₃ einen pH-Wert von 5.5 bis 6.0 ein und zieht dann am Rotavapor bei maximal 45 °C das Wasser unter reduziertem Druck ab. Der feste Rückstand wird aus Methanol/Acton kristallisiert. Man erhält **9e** in Form beiger Nadeln in guter Ausbeute.

### Beispiel 10: 1-N-(2-Phosphonoethyl)-3-hydroxy-7-methyl-xanthin (10)

### 1-N-(2-Dimethylphosphono-ethyl)-7-methylxanthin (10a)

50 g 7-Methylxanthin (2,6-Dihydroxy-7-methylpurin) werden in 1 l siedendem Ethanol gelöst und 38 g 50%ige Kalilauge dazu gegeben. Nach dem Kühlen auf 15 bis 20 °C fällt das Kaliumsalz von 7-Methylxanthin aus, welches abfiltriert und mit siedendem Aceton sowie siedendem absolutem Ethanol ausgekocht wird.

Eine Lösung von 20 mmol 2-Bromethylphosphonsäuredimethylester und 2 mmol Hexadecyltributyl-phosphoniumbromid in 10 ml Toluol, versetzt man mit 25 mmol des Kaliumsalzes von 7-Methylxanthin und erhitzt 2 h auf 100 °C. Nach Abkühlen der Reaktionsmischung filtriert man von Ungelöstem ab und chromatographiert die eingeengte organische Phase an Kieselgel mit Ether/Chloroform als Laufmittel. Auf diese Weise erhält man sowohl das gewünschte 1-N-(2-Dimethylphosphono-ethyl)-7-methylxanthin (**10a**) als auch in geringerer Ausbeute 3-N-(2-Dimethylphosphono-ethyl)-7-methylxanthin (**10a'**).

### 1-N-(2-Dimethylphosphono-ethyl)-3-hydroxy-7-methyl-xanthin (10b)

Zu einer auf 0 °C gekühlten Lösung von 25 mmol **10a** in 50 ml trockenem Aceton wird eine Lösung von 60 mmol Dimethyldioxiran in 120 ml trockenem Aceton getropft. Man läßt 30 min bei 0 °C rühren und zieht dann das Lösungsmittel unter reduziertem Druck ab. Das erhaltene Rohprodukt wird an Kieselgel chromatographiert, wobei man in schlechter Ausbeute 1-N-(2-Dimethylphosphono-ethyl)-3-hydroxy-7-methyl-xanthin (**10b**) erhält.

Analog dazu kann 3-N-(2-Dimethylphosphonoethyl)-7-methylxanthin (**10a'**) zu 3-N-(2-Dimethylphosphono-ethyl)-1-hydroxy-7-methylxanthin (**10b'**) umgesetzt werden.

### 1-N-(2-Phosphono-ethyl)-3-hydroxy-7-methyl-xanthin (10c)

Zu 25 mmol **10b** in 50 ml absolutiertem Acetonitril tropft man unter Eiskühlung 4 Äquivalente (100 mmol) Trimethylbromsilan, rührt 15 min bei gleicher Temperatur, dann 2 h bei RT, engt unter reduziertem Druck bis zu einem Öl ein, nimmt in 100 ml Wasser auf und hydrolysiert 1 h bei RT. Diese Lsg. wird zum Entfernen von Hexamethyldisiloxan zweimal mit CHCl₃ extrahiert, einmal mit Wasser zurück extrahiert und die vereinigten wäßrigen Phasen unter reduziertem Druck bei 45 °C eingeengt. Das entstehende beige Öl wird in Wasser aufgenommen und ein pH von 6.5 - bis 7.0 eingestellt. **10c** fällt nach Waschen mit Eiswasser als nahezu farbloses Natriumsalz in 55 %iger Ausbeute an.

Analog dazu wird 3-N-(2-Dimethylphosphono-ethyl)-1-hydroxy-7-methyl-xanthin (**10b**') mit Trimethylbromsilan zu 3-N-(2-Phosphono-ethyl)-1-hydroxy-7-methyl-xanthin (**10c**') umgesetzt.

### Beispiel 11: N-Hydroxy-1,2,3,4-tetrahydro-1-oxo-3-[2-phosphonoethyl)]-isochinolin (11)

### 3-Phenyl-2-aminopropanol (11a)

In einem ausgeheizten und mit Argon gefluteten Dreihalskolben mit KPG-Rührer und Metallkühler werden 3.0 mol LiAlH₄ in 900 ml wasserfreiem Tetrahydrofuran suspendiert und unter Eiskühlung portionsweise 1.5 mol Phenylalanin zugegeben. Danach erhitzt man 6 h unter Rückfluß, läßt abkühlen und hydrolysiert mit zerstoßenem Eis. Es wird filtriert und das Lösungsmittel im Vakuum entfernt. Das Filtrat wird mit CH₂Cl₂ aufgenommen, mit gesättigter NaCl-Lösung gewaschen und mit Na₂SO₄ getrocknet. Anschließend wird im Vakuum destilliert. Man erhält 3-Phenyl-2-aminopropanol **11a** in einer Ausbeute von 76 %.

### 1-Phenyl-3-(tetrahydro-2-pyranyloxy)-2-aminopropan (11b)

Zu 1.4 mol 3-Phenyl-2-aminopropanol **1** werden 2.5 mol Dihydropyran und 5.3 g p-Toluolsulfonsäure gegeben und anschließend 20 h bei RT gerührt. Danach wird das überschüssige Dihydropyran im Vakuum entfernt, der Rückstand mit 700 ml Ethylacetat aufgenommen und mit je 300 ml gesättigter NaHCO₃-Lösung und gesättigter NaCl-Lösung gewaschen. Anschließend wird mit MgSO₄ getrocknet, filtriert und das Lösungsmittel im Vakuum entfernt. Man erhält in 63 %iger Ausbeute 1-Phenyl-3-(tetrahydro-2-pyranyloxy)-2-aminopropan (**11b**).

### 1-Phenyl-3-(tetrahydro-2-pyranyloxy)-2-isocyanopropan (11c)

Zu einer Lösung von 3.52 mol Phosgen in 1.5 I Toluol werden bei RT 0.88 mol 1-Phenyl-3-(tetrahydro-2-pyranyloxy)-2-aminopropan **11b** hinzugetropft und danach 3 h bei 80°C gekocht. Anschließend wird das Lösungsmittel im Vakuum entfernt. Man erhält in einer Ausbeute von 83 % das gewünschte Produkt 1-Phenyl-3-(tetrahydro-2-pyranyloxy)-2-isocyanopropan **11c.** Dieses wird ohne weitere Reinigung weiter verwendet.

### 1,2,3,4-Tetrahydro-1-oxo-3-hydroxymethyl-isochinolin (11d)

Eine Lösung von 0.72 mol 1-Phenyl-3-(tetrahydro-2-pyranyloxy)-2-isocyanopropan **11c** in 80 ml wasserfreiem Aceton wird langsam zu 100 ml eisgekühlter Phosphorsäure hinzugetropft und 3 h bei RT gerührt. Anschließend wird Eiswasser zugesetzt, 0.5 h gerührt und dann mit CH₂Cl₂ extrahiert. Die organische Phase wird mit Wasser, gesättigter Na₂CO₃-Lösung, wieder mit Wasser und mit gesättigter NaCl-Lösung gewaschen und mit MgSO₄ getrocknet. Nach Filtration und Entfernen des Lösungsmittels im Vakuum wird aus Hexan/Benzol umkristallisiert. Man erhält 28 % 1,2,3,4-Tetrahydro-1-oxo-3-hydroxymethyl-isochinolin (**11d**).

### 1,2,3,4-Tetrahydro-1-oxo-3-brommethyl-isochinolin (11e)

Eine Lösung von 180 mmol PPh₃ in 120 ml CH₂Cl₂ wird zu einer Lösung von 150 mmol 1,2,3,4-Tetrahydro-1-oxo-3-hydroxymethyl-isochinolin **11c** und 210 mmol CBr₄ in 150 ml CH₂Cl₂ gegeben und 20 h bei RT gerührt. Dann wird das Lösungsmittel im Vakuum entfernt und der Rückstand mehrfach aus Benzol umkristallisiert. Man erhält das Produkt 1,2,3,4-Tetrahydro-1-oxo-3-brommethyl-isochinolin **11e** in einer Ausbeute von 31 %.

### 1,2,3,4-Tetrahydro-1-oxo-3-(2-diethylphosphonoethyl)-isochinolin (11f)

Zu einer Lösung von 75 mmol Dimethylmethylphosphonat in 120 ml absolutem THF unter Argon werden bei -78°C 66.2 mmol einer Lösung von n-Butyllithium (1.15 M) in Hexan getropft und 1.5 h bei dieser Temperatur gerührt. Zu dieser Lösung werden bei -78°C 46.5 mmol 1,2,3,4-Tetrahydro-1-oxo-3-brommethyl-isochinolin **11e** in 50 ml absolutem THF getropft, noch 1 h bei -78°C gerührt und dann über Nacht auf RT kommen lassen. Nun werden 100 ml Wasser zugegeben, die wäßrige Phase abgetrennt und 3 mal mit je 50 ml Ethylacetat extrahiert. Die vereinigten organischen Phasen werden mit MgSO₄ getrocknet, filtriert und das Lösungsmittel im Vakuum entfernt. Der Rückstand wird chromatographisch (Kieselgel, Hexan/Ethylacetat 5 : 1) gereinigt. Man erhält 24 % des gewünschten Produktes 1,2,3,4-Tetrahydro-1-oxo-3-(2-diethylphosphonoethyl)-isochinolin **11f.**

### N-Hydroxy-1,2,3,4-tetrahydro-1-oxo-3-(2-diethylphosphonoethyl)-isochinolin (11g)

5.36 mmol 1,2,3,4-Tetrahydro-1-oxo-3-(2-diethylphosphonoethyl)-isochinolin **6** werden in 30 ml absolutem Aceton gelöst und auf 0°C gekühlt. Dann wird eine Lösung von 17.15 mmol Dimethyldioxiran hinzugetropft und 30 Min. bei 0°C gerührt. Das Lösungsmittel wird im Vakuum entfernt und der Rückstand chromatographisch gereinigt (Kieselgel, Hexan/Ethylacetat 4 : 1). Man erhält 33 % des Produktes
N-Hydroxy-1,2,3,4-tetrahydro-1-oxo-3 -(2-diethylphosphonoethyl)-isochinolin **11g.**

### N-Hydroxy-1,2,3,4-tetrahydro-1-oxo-3-(2-phosphonoethyl)-isochinolin (11h)

In 15 ml absolutem CH₂Cl₂ werden unter Argon 1.77 mmol N-Hydroxy-1,2,3,4-tetrahydro-1-oxo-3-(2-diethylphosphonoethyl)-isochinolin **11g** gelöst und auf 0°C gekühlt. Jetzt werden aus einer Spritze 10 mmol Trimethylbromsilan zugetropft, noch 1 h bei 0°C und dann über Nacht bei RT gerührt. Nun wird das Lösungsmittel im Vakuum entfernt, der Rückstand in 20 ml Wasser aufgenommen und 1 h bei RT gerührt. Anschließend werden 15 ml CHCl₃ zugesetzt und die organische Phase abgetrennt. Die wäßrige Phase wird noch 2 mal mit je 10 ml CHCl₃ extrahiert und dann im Vakuum eingedampft. Der Rückstand wird chromatographisch gereinigt (Kieselgel, H₂O/Methanol 1 : 1). Man erhält das gewünschte Produkt in einer Ausbeute von 54 %.

### Beispiel 12:

Es wurde die Antimalaria-Wirksamkeit für die in Tabelle I angegebenen Substanzen an invitro-Kulturen des Malaria-Erregers Plasmodium falciparum bestimmt. Die Angaben in römischen Ziffern beziehen sich auf die auf den Seiten bis angegebenen besonders bevorzugten Verbindungen. Die Vertiefungen einer 96-well- Mikrotiterplatte wurden mit je 200 µl einer asynchronen Plasmodium falciparum-Kultur bei 0,4 % Parasitämie und 2 % Hämatokrit beschickt. Dann wurde eine serielle Verdünnungsreihe der Verbindungen in Dreierschritten zwischen Konzentrationen von 100 bis 1 µmol l⁻¹ hergestellt. Die Platten werden bei 37°C, 3 % CO₂ und 5 % O₂ über einen Zeitraum von 48 Stunden inkubiert. Dann wurden zu jedem well 30 µl Medium supplementiert mit 27 µCi ml⁻¹ [³H]-Hypoxanthin zugefügt. Nach 24-stündiger Inkubation wurden die Parasiten durch Filtration auf Glasfaserfilter geerntet und die incorporierte Radioaktivität gemessen. Die Inhibition des Parasitenwachstums wurde als prozentuale Inhibition der Tritiumcorporation bezogen auf einen Vergleich ohne Substanz ausgedrückt. Die Ergebnisse sind für die drei verschiedenen Konzentrationen in Tabelle I angeben.

**Tabelle 1**

| **Substanz** | **R**_{**5**} | **A** | **eingesetzte Form** | **100 µM Inhibition (%)** | **10 µM Inhibition (%)** | **1 µM Inhibition (%)** |
|---|---|---|---|---|---|---|
| **I** | H | C₂H₄ | Na-Salz | 98 | 98 | 98 |
| **III** | H | CH₂ | Na-Salz | 96 | 98 | 63 |
| **II** | H | CH₂ | Na-Salz | 98 | 87 | 59 |
| **I** | H | CH₂CHOH | Na-Salz | 98 | 97 | 78 |
| **X** | H | CH₂ | Na-Salz | 89 | 80 | 0 |
| **X** | H | CH₂ | Na-Salz | 98 | 97 | 98 |
| **III** | H | CH₂ | Na-Salz Pyrrolring ist in 3-Stellung mit NH₂ substituiert | 98 | 98 | 82 |
| **VII** | H | C₂H₄ | Na-Salz | 98 | 98 | 98 |
| **XXXI** | H | C₂H₄ | Na-Salz | 97 | 73 | 53 |
| **XXVIII** | H | C₂H₄ | Na-Salz | 98 | 97 | 82 |
| **XXXVIII** | H | C₂H₄ N-substituiert in 5-Stellung | Na-Salz | 92 | 78 | 30 |
| **XXXVI** | H | C₂H₄ in 3-Stellung | Na-Salz | 95 | 80 | 37 |

### Beispiel 13

Es wurde die antibakterielle Wirkung für die in Tabelle II angegebenen Substanzen bestimmt. Die Angaben in römischen Ziffern beziehen sich auf die auf den Seiten 5 bis 8 angegebenen besonders bevorzugten Verbindungen.
In 5 Kulturröhrchen wurde eine Verdünnungsserie mit den Konzentrationen 500, 100, 50, 10 und 0 µmol l⁻¹ der einzelnen Verbindungen in LB-Medium in einem Volumen von 0,5 ml vorgelegt. Die Röhrchen wurden mit je 10 µl aus der Übernachtkultur von E. coli K12 inokuliert und über Nacht bei 37°C geschüttelt. Das Wachstum der Bakterien wurde durch Trübung des Mediums beurteilt. Es wurde die minimale Konzentration bestimmt, bei der das bakterienwachstum inhibiert wurde (Minimale Inhibitionskonzentration MIC)

Die antibakterielle Wirksamkeit von P. aeruginosa wurde gleichermaßen bestimmt. Die Ergebnisse sind in Tabelle II aufgeführt.

**Tabelle II**

| **Substanz** | **R**_{**5**} | **A** | **eingesetzte Form** | **E.coli MIC (mg/l)** | **P. aeruginosa mg/l** |
|---|---|---|---|---|---|
| **I** | H | C₂H₄ | Na-Salz | 2,5 | 5 |
| **III** | H | CH₂ | Na-Salz Pyrrolring ist in 3-Stellung mit NH₂ substituiert | 5 | 5 |
| **II** | H | CH₂ | Na-Salz | 10 | 5 |
| **I** | H | CH₂CHOH | Na-Salz | 2,5 | 1,25 |
| **X** | H | CH₂ | Na-Salz | 1,25 | 1,25 |
| **X** | H | CH₂ | Na-Salz | 10 | 20 |
| **III** | H | CH₂ | Na-Salz | 1,25 | 10 |
| **VII** | H | C₂H₄ | Na-Salz | 10 | 40 |
| **XXXI** | H | C₂H₄ | Na-Salz | 10 | 1,25 |
| **XXVIII** | H | C₂H₄ | Na-Salz | 5 | 5 |
| **XXXVIII** | H | C₂H₄ N-substituiert in 5-Stellung | Na-Salz | 20 | 80 |
| **XXXVI** | H | C₂H₄ in 3-Stellung | Na-Salz | 40 | 20 |

## Patentansprüche

1. Phosphororganische Verbindungen der allgemeinen Formel (I) 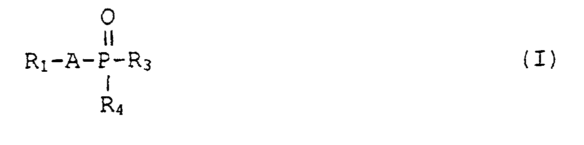 wobei A aus der Gruppe ausgewählt ist, die aus einem (C₁₋₉)-Alkylenrest, der ein oder mehrere Doppelbindungen aufweisen kann und mit Hydroxy-, Halogen-, Amino-, Oxogruppen mit verzweigten oder unverzweigten C₁₋₉-Alkylgruppen und C₂₋₉-Alkenylgruppen substituiert sein kann, wobei die C₁₋₉-Alkylgruppen und C₂₋₉-Alkenylgruppen mit Wasserstoff-, Hydroxy-, Amino-, Halogen- und Oxogruppen substituiert sein können, -C-O-C- und -C-N-C- besteht, wobei die Kohlenstoffatome von -C-O-C- und -C-N-C- mit einem Alkyl mit bis zu 7 Kohlenstoffatomen oder Hydroxygruppen substituiert sein können.
oder in der A der folgenden Formel (II) entspricht: 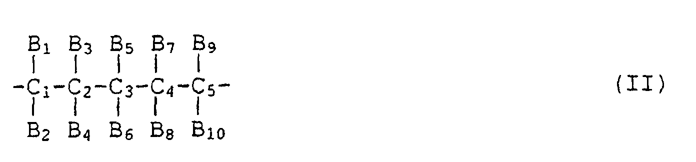 wobei ein oder mehrere der Kohlenstoffatome, ausgewählt aus der Gruppe C₃, C₄, C₅, mitsamt ihren Substituenten auch wegfallen können, und mindestens ein vorliegender Substituent von B₁ bis B₁₀ eine C₃₋₈-Cycloalkyl-(C₀₋₉)-alkylgruppe ist, wobei sowohl die C₃₋₈-Cycloalkylgruppe als auch die C₀₋₉-Alkylgruppe ein oder mehrere Doppelbindungen aufweisen können und ein oder zwei Kohlenstoffatome der Cycloalkylgruppe durch Stickstoff-, Sauerstoff- oder Schwefelatome ersetzt sein können, und wobei sowohl die Cycloalkylgruppe als auch die Alkylgruppe mit Hydroxy-, Halogen-, Amino-, Oxogruppen mit verzweigten oder unverzweigten C₁₋₉-Alkylgruppen und C₂₋₉-Alkenyigruppen substituiert sein können, wobei die C₁₋₉-Alkylgruppen und C₂₋₉-Alkenylgruppen mit Wasserstoff-, Hydroxy-, Amino-, Halogen- und Oxogruppen substituiert sein können, und die restlichen vorliegenden Substituenten B₁ bis B₁₀ aus der Gruppe ausgewählt sind, die aus Wasserstoff, Hydroxy-, Halogen-, Aminogruppen, C₁₋₂₆-Alkylresten, C₁₋₂₆-Alkoxyresten, C₁₋₂₆-Alkoxy-C₁₋₂₆-Alkylresten besteht oder beide Substituenten eines C-Atoms zusammen eine Oxogruppe bilden, wobei jeder C₁₋₂₆-Alkylrest und jeder C₁₋₂₆-Alkoxyrest verzweigt oder unverzweigt und gesättigt oder mit ein oder mehreren Doppelbindungen ungesättigt sein kann und mit Hydroxy-, Amino-, Halogen- und Oxogruppen substituiert sein kann,
in der R₁ aus der Gruppe ausgewählt ist, die aus 5- und 6-gliedrigen Heterocyclen mit mindestens einem Stickstoffatom im Ring oder polycyclischen Kohlenwasserstoffen, die mindestens einen dieser Heterocyclen enthalten, besteht, wobei mindestens eines dieser Stickstoffatome zu einer Hydroxamsäuregruppe oder Hydroxamsäureestergruppe gehört, und gesättigt oder mit ein oder mehreren Doppel- oder Dreifachbindungen ungesättigt und damit auch aromatisch sein kann und mit Hydroxy-, Halogen-, Amino-, Oxogruppen und mit verzweigten oder unverzweigten C₁₋₉-Alkylgruppen und C₂₋₉-Alkenylgruppen substituiert sein kann, wobei die C₁₋₉-Alkylgruppen und C₂₋₉-Alkenylgruppen gesättigt oder mit ein oder mehreren Doppel- oder Dreifachbindungen ungesättigt sein können und mit Wasserstoff-, Hydroxy-, Amino-, Halogen- und Oxogruppen substituiert sein können und wobei das Stickstoffatom der Hydroxamsäuregruppe oder -säureestergruppe mit OR₅ substiuiert ist und
R₅ aus der Gruppe ausgewählt ist, die aus Wasserstoff, substituiertem und unsubstituiertem C₁₋₉-Alkyl, substituiertem und unsubstituiertem Hydroxy-C₁₋₉-alkyl, substituiertem und unsubstituiertem C₂₋₉-Alkenyl, substituiertem und unsubstituiertem C₂₋₉-Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest besteht,
in der R₃ und R₄ gleich oder verschieden sind und aus der Gruppe ausgewählt sind, die aus Wasserstoff, substituiertem und unsubstituiertem C₁₋₂₆-Alkyl, Hydroxy-C₁₋₂₆-alkyl, substituiertem und unsubstituiertem Aryl, substituiertem und unsubstituiertem Acyl, substituiertem und unsubstituiertem Aralkyl, substituiertem und unsubstituiertem C₂₋₂₆-Alkenyl, substituiertem und unsubstituiertem C₂₋₂₆-Alkinyl, substituiertem und unsubstituiertem Cycloalkyl, substituiertem und unsubstituiertem heterocyclischem Rest, Halogen, OX₃ und OX₄ besteht,
wobei X₃ und X₄ gleich oder verschieden sind und aus der Gruppe ausgewählt sind, die aus Wasserstoff, substituiertem und unsubstituiertem C₁₋₂₆-Alkyl, substituiertem und unsubstituiertem Hydroxy-C₁₋₂₆-alkyl, substituiertem und unsubstituiertem Aryl, substituiertem und unsubstituiertem Aralkyl, substituiertem und unsubstituiertem C₂₋₂₆-Alkenyl, substituiertem und unsubstituiertem C₂₋₂₆-Alkinyl, substituiertem und unsubstituiertem Cycloalkyl, substituiertem und unsubstituiertem heterocyclischem Rest, einem Silyl, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht,
und deren pharmazeutisch akzeptablen Salze, Ester und Amide und Salze der Ester.

2. Verbindung nach Anspruch 1, **dadurch gekennzeichnet, daß** die phosphororganischen Verbindungen der Formel (III) 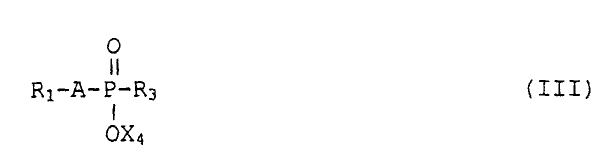 entspricht, wobei R₃ bevorzugt Wasserstoff, Methyl, Ethyl oder ein Amidrest ist und X₄ aus der Gruppe ausgewählt ist, die aus Wasserstoff, Natrium, Kalium, Methyl, Ethyl besteht.

3. Verbindung nach Anspruch 1, **dadurch gekennzeichnet, daß** die phosphororganischen Verbindungen der Formel (IV) 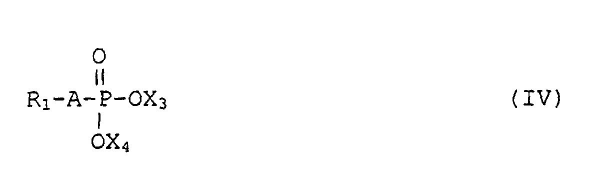 entspricht, wobei X₃ und X₄ gleich oder verschieden und aus der Gruppe ausgewählt sind, die aus Wasserstoff, einem (C₁₋₃)-Alkyl, einem Metall der ersten, zweiten oder dritten Hauptgruppe des Periodensystems, Ammonium, substituiertem Ammonium, oder Ammoniumverbindungen, die sich von Ethylendiamin oder Aminosäuren ableiten, besteht.

4. Verbindung nach Anspruch 3, **dadurch gekennzeichnet, daß** X₃ und X₄ gleich oder verschieden sind und aus der Gruppe ausgewählt sind, die aus Wasserstoff, Natrium, Kalium, Methyl, Ethyl besteht.

5. Verbindung nach einem der vorhergehenden Ansprüche, **dadurch gekennzeichnet, daß** A aus der Gruppe ausgewählt ist, die aus Alkylen, Alkenylen, Hydroxyalkylen und Oxoalkylen besteht.

6. Verbindung nach Anspruch 5, **dadurch gekennzeichnet, daß** A so ausgewählt ist, daß zwischen dem Stickstoffatom der heterocyclische Gruppe und das Phosphoratom drei Atome vorliegen, wobei A bevorzugt ein Methylen, Hydroxymethylen, Ethylen, Ethenylen oder Hydroxyethylen ist.

7. Verbindung nach einem der Ansprüche 1 bis 3, **dadurch gekennzeichnet, daß** die Verbindung aus der Gruppe ausgewählt ist, die aus 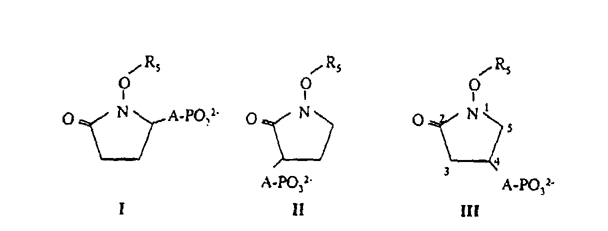 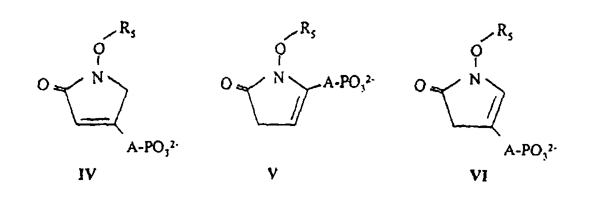 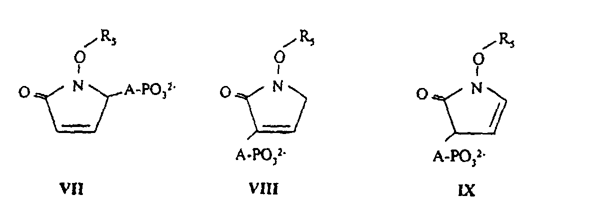 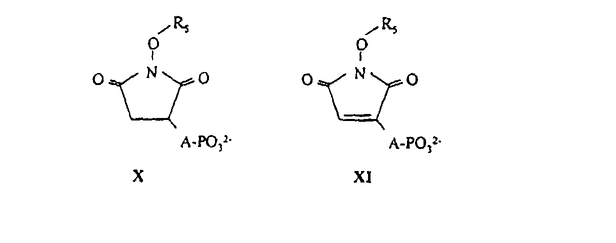 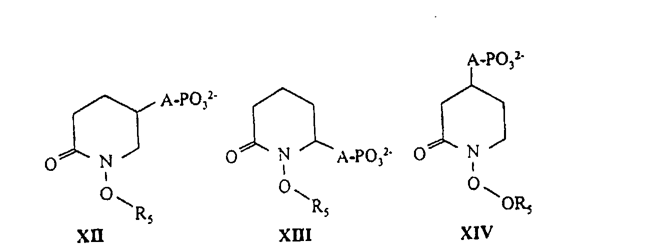 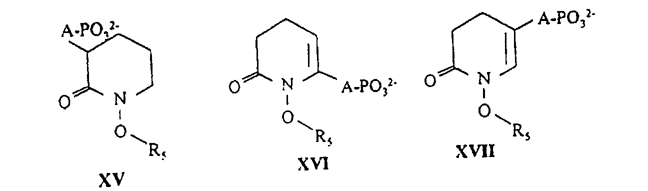 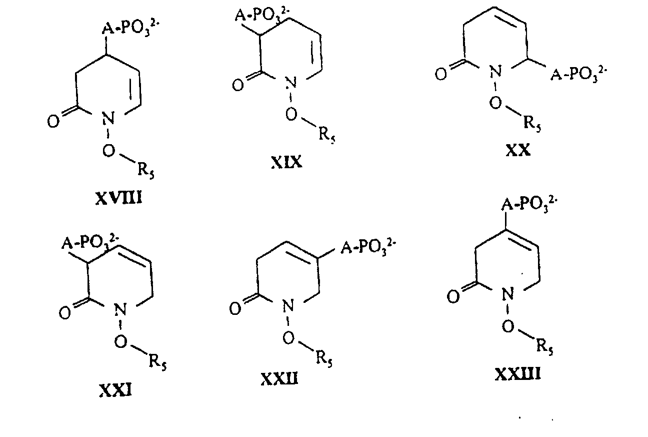 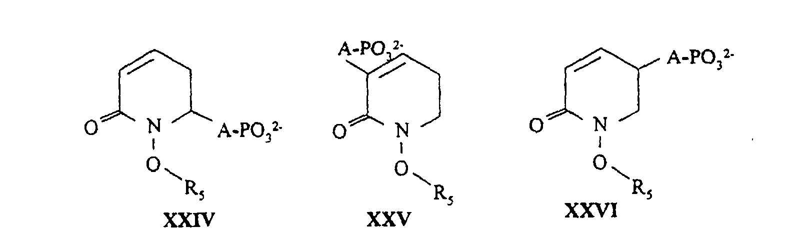 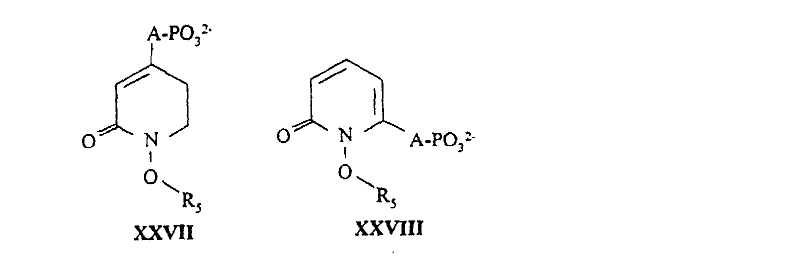 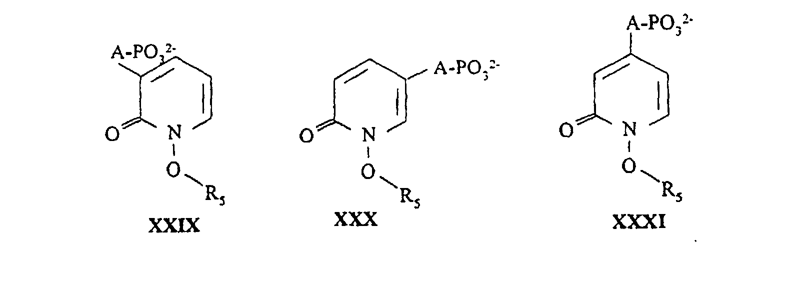 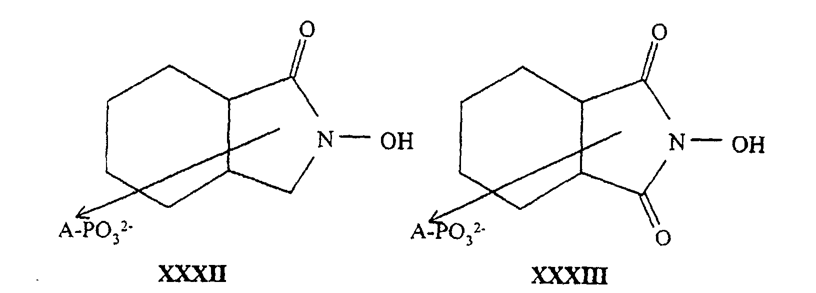 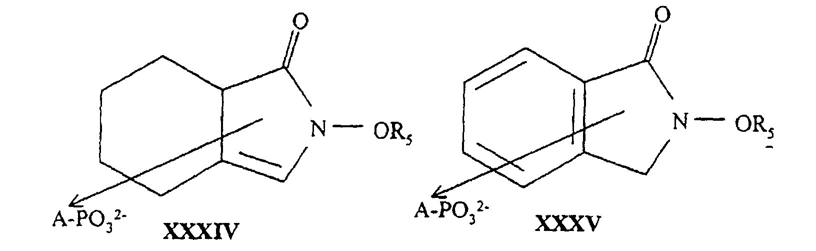 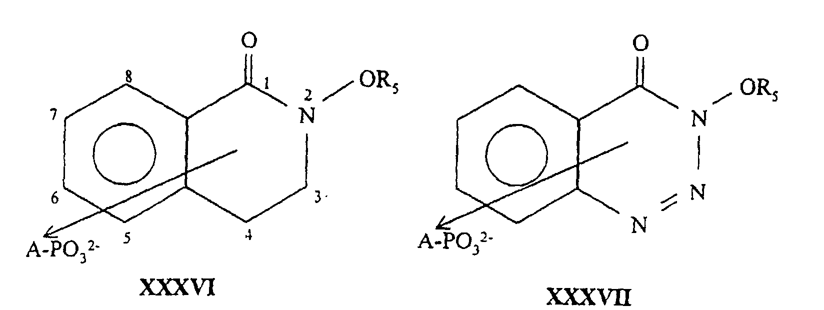 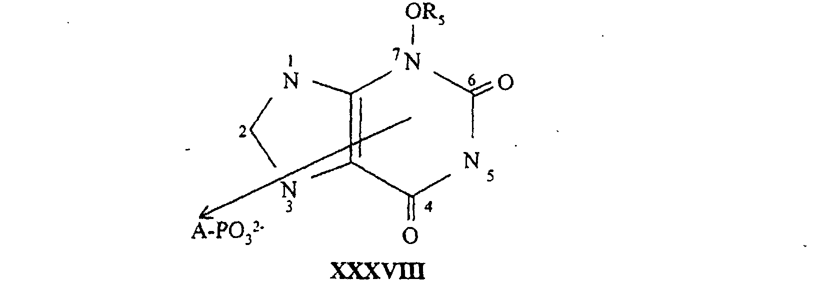 und den entsprechenden Phosphinsäure- und Phosphinoylderivaten besteht, wobei R₅ wie in Anspruch 1 definiert ist.

8. Verwendung einer Verbindung nach einem der Ansprüche 1 bis 7 als Fungizid, Bakterizid oder Herbizid bei Pflanzen.

9. Verwendung einer Verbindung nach einem der Ansprüche 1 bis 7 zur Behandlung von Infektionen, verursacht durch Bakterien, Viren, Pilze oder ein- oder mehrzellige Parasiten.

10. Verwendung nach Anspruch 9 zur Vorbeugung und Behandlung von Infektionen verursacht durch einzellige Parasiten, nämlich Erreger der Malaria, der Schlafkrankheit, der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akantha-möbose, der Naeglerose, der Kokzidiose, der Giardiose und der Lambliose.

11. Pharmazeutisches Präparat zur therapeutischen und prophylaktischen Behandlung infektiöser Prozesse, **dadurch gekennzeichnet, daß** das Präparat einen wirksamen Gehalt an zumindest einer phosphororganischen Verbindung nach einem der Ansprüche 1 bis 7 zusammen mit einem pharmazeutisch akzeptablen Träger enthält.

12. Pharmazeutisches Präparat nach Anspruch 11, **dadurch gekennzeichnet, daß** das Präparat einen weiteren pharmazeutischen Wirkstoff enthält.

## Claims

1. Organophosphorus compounds of the general formula (I) 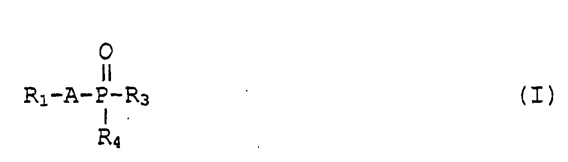 wherein A is selected from the group which consists of a (C₁₋₉) alkylene residue, which may comprise one or more double bonds and may be substituted with hydroxy, halogen, amino, oxo groups with branched or unbranched C₁₋₉ alkyl groups and C₂₋₉ alkenyl groups, wherein the C₁₋₉ alkyl groups and C₂₋₉ alkenyl groups may be substituted with hydrogen, hydroxy, amino, halogen and oxo groups, -C-O-C- and -C-N-C-, wherein the carbon atoms of -C-O-C- and -C-N-C- may be substituted with an alkyl having up to 7 carbon atoms or hydroxy groups,
or in which A is of the following formula (II): 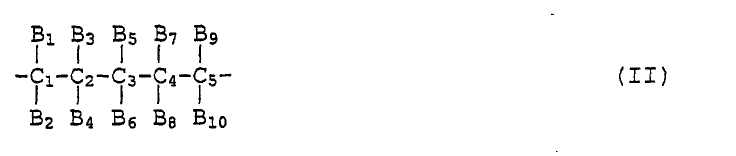 wherein one or more of the carbon atoms selected from the group C₃, C₄, C₅, together with their substituents, may also be absent, and at least one substituent present in the range from B₁ to B₁₀ is a C₃₋₈-cycloalkyl-(C₀₋₉)-alkyl group, wherein both the C₃₋₈ cycloalkyl group and the C₀₋₉ alkyl group may comprise one or more double bonds and one or two carbon atoms of the cycloalkyl group may be replaced by nitrogen, oxygen or sulfur atoms, and wherein both the cycloalkyl group and the alkyl group may be substituted with hydroxy, halogen, amino, oxo groups with branched or unbranched C₁₋₉ alkyl groups and C₂₋₉ alkenyl groups, wherein the C₁₋₉ alkyl groups and C₂₋₉ alkenyl groups may be substituted with hydrogen, hydroxy, amino, halogen and oxo groups, and the remaining substituents B₁ to B₁₀ present are selected from the group which consists of hydrogen, hydroxy, halogen, amino groups, C₁₋₂₆ alkyl residues, C₁₋₂₆ alkoxy residues, C₁₋₂₆-alkoxy-C₁₋₂₆-alkyl residues or both substituents of a C atom together form an oxo group, wherein each C₁₋₂₆ alkyl residue and each C₁₋₂₆ alkoxy residue may be branched or unbranched and be saturated or unsaturated with one or more double bonds and may be substituted with hydroxy, amino, halogen and oxo groups,
in which R₁ is selected from the group which consists of 5- and 6-membered heterocycles with at least one ring nitrogen atom or a polycyclic carbon with at least one of these heterocycles, wherein at least one of these nitrogen atoms belongs to a hydroxamic acid group or a hydroxamic acid ester group, and may be saturated or unsaturated with one or more double or triple bonds and may thus also be aromatic and may be substituted with hydroxy, halogen, amino, oxo groups and with branched or unbranched C₁₋₉ alkyl groups and C₂₋₉ alkenyl groups, wherein the C₁₋₉ alkyl groups and C₂₋₉ alkenyl groups may be saturated or unsaturated with one or more double or triple bonds and may be substituted with hydrogen, hydroxy, amino, halogen and oxo groups,
wherein the nitrogen atom of the hydroxamic acid group or hydroxamic acid ester group is substituted with OR₅ and
R₅ is selected from the group which consists of hydrogen, substituted and unsubstituted C₁₋₉ alkyl, substituted and unsubstituted hydroxy-C₁₋₉-alkyl, substituted and unsubstituted C₁₋₉ alkenyl, substituted and unsubstituted C₁₋₉ alkynyl, substituted and unsubstituted aryl, substituted and unsubstituted acyl, substituted and unsubstituted cycloalkyl, substituted and unsubstituted aralkyl, substituted and unsubstituted heterocyclic residue,
in which R₃ and R₄ are identical or different and are selected from the group which consists of hydrogen, substituted and unsubstituted C₁₋₂₆ alkyl, hydroxy-C₁₋₂₆-alkyl, substituted and unsubstituted aryl, substituted and unsubstituted acyl, substituted and unsubstituted aralkyl, substituted and unsubstituted C₁₋₂₆ alkenyl, substituted and unsubstituted C₁₋₂₆ alkynyl, substituted and unsubstituted cycloalkyl, substituted and unsubstituted heterocyclic residue, halogen, OX₃ and OX₄,
wherein X₃ and X₄ are identical or different and are selected from the group which consists of hydrogen, substituted and unsubstituted C₁₋₂₆ alkyl, substituted and unsubstituted hydroxy-C₁₋₂₆-alkyl, substituted and unsubstituted aryl, substituted and unsubstituted aralkyl, substituted and unsubstituted C₁₋₂₆ alkenyl, substituted and unsubstituted C₁₋₂₆ alkynyl, substituted and unsubstituted cycloalkyl, substituted and unsubstituted heterocyclic residue, a silyl, a cation of an organic and inorganic base, in particular a metal of main groups I, II or III of the periodic system, ammonium, substituted ammonium and ammonium compounds derived from ethylenediamine or amino acids,
and the pharmaceutically acceptable salts, esters and amides thereof and salts of the esters.

2. Compound according to claim 1, **characterised in that** the organophosphorus compounds are of the formula (III) 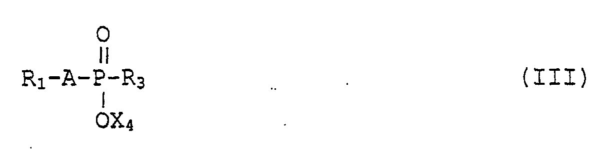 wherein R₃ is preferably hydrogen, methyl, ethyl, an amide residue and X₄ is selected from the group which consists of hydrogen, sodium, potassium, methyl, ethyl.

3. Compound according to claim 1, **characterised in that** the organophosphorus compounds are of the formula (IV) 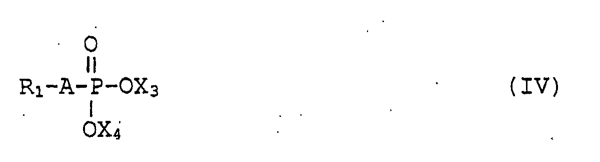 wherein X₃ and X₄ are identical or different and are selected from the group which consists of hydrogen, a (C₁₋₃) alkyl, a metal of main groups I, II or III of the periodic system, ammonium, substituted ammonium, or ammonium compounds derived from ethylenediamine or amino acids.

4. Compound according to claim 3, **characterised in that** X₃ and X₄ are identical or different and are selected from the group which consists of hydrogen, sodium, potassium, methyl, ethyl.

5. Compound according to one of the preceding claims, **characterised in that** A is selected from the group which consists of alkylene, alkenylene, hydroxyalkylene and oxoalkylene.

6. Compound according to claim 5, **characterised in that** A is selected such that three atoms are present between the nitrogen atom of the heterocyclic group and the phosphorus atom, wherein A is preferably a methylene, hydroxymethylene, ethylene, ethenylene or hydroxyethylene.

7. Compound according to one of claims 1 to 3, **characterised in that** the compound is selected from the group of compounds which consists of 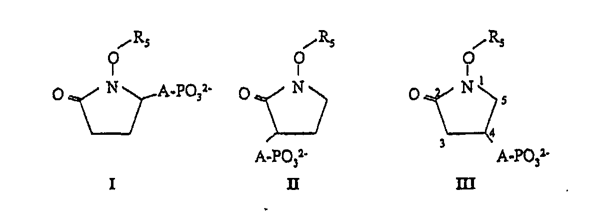 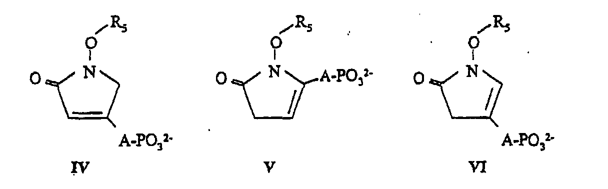 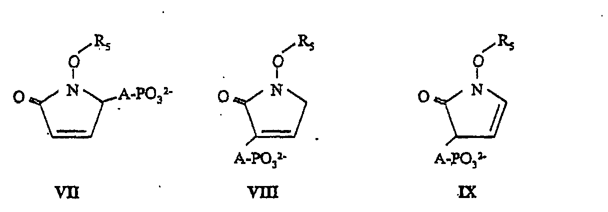 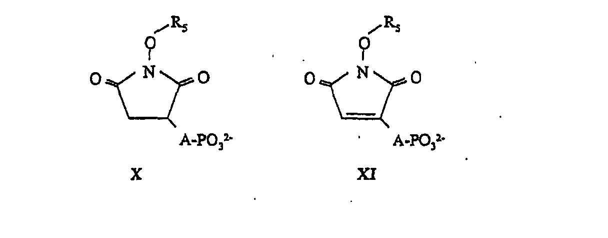 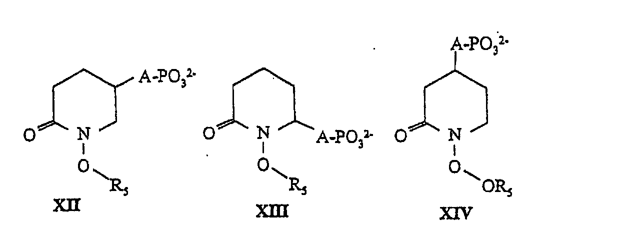 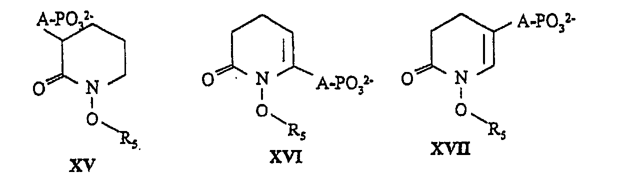 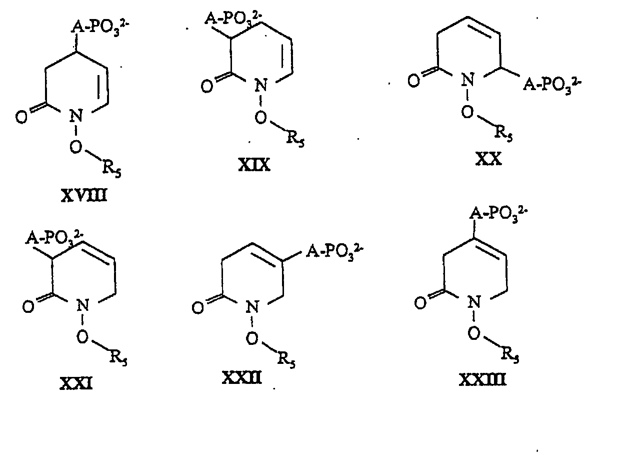 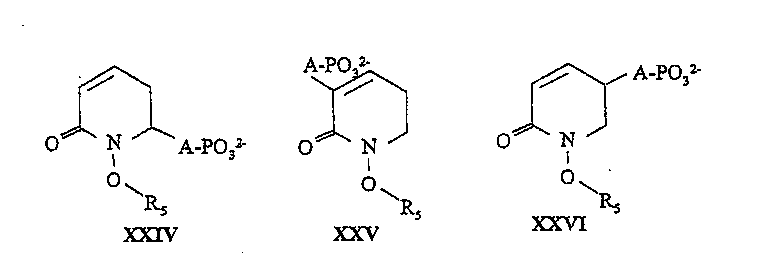 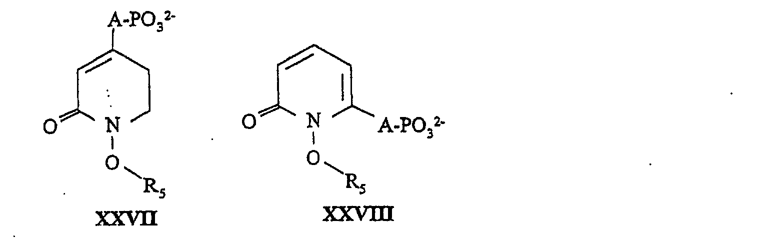 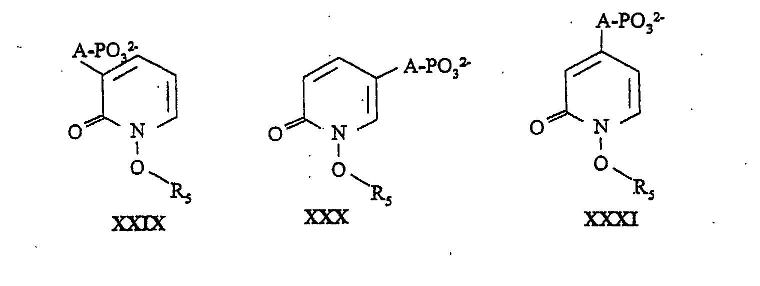 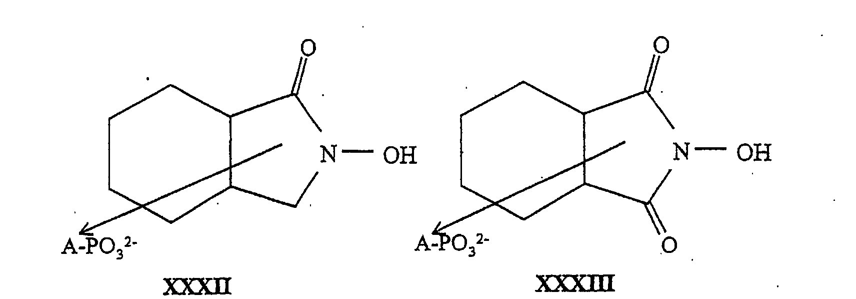 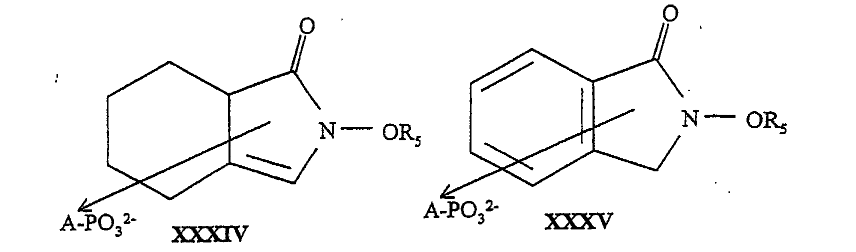 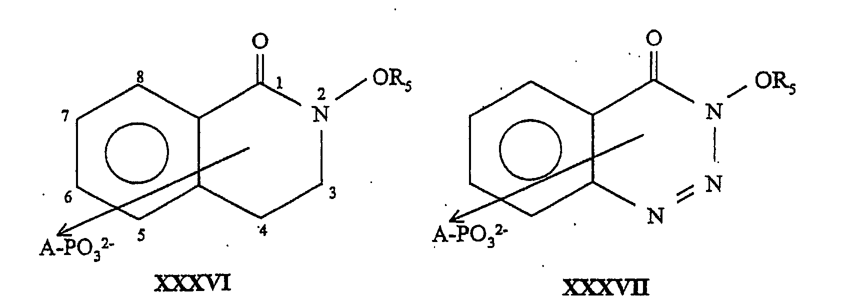 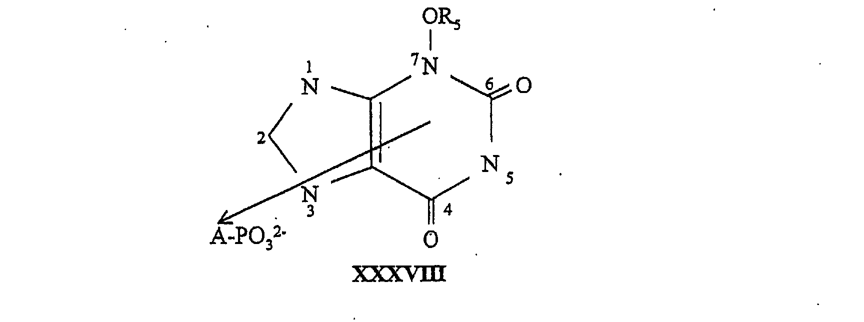 and the corresponding phosphinic acid and phosphinoyl derivatives, wherein R₅ is defined as in claim 1.

8. Use of a compound according to one of claims 1 to 7 as a fungicide, bactericide or herbicide in plants.

9. Use according to one of claims 1 to 7 for the treatment of infections caused by bacteria, viruses, fungi or uni- or multicellular parasites.

10. Use according to claim 9 for the prevention and treatment of infections caused by unicellular parasites, namely the causative organisms of malaria, sleeping sickness, Chagas' disease, toxoplasmosis, amoebic dysentery, leishmaniases, trichomoniasis, pneumocystosis, balantidiasis, cryptosporidiosis, sarcocytosis, acanthamoebosis, naeglerosis, coccidiosis, giardiasis and lambliasis.

11. Pharmaceutical preparation for the therapeutic and prophylactic treatment of infectious processes, **characterised in that** the preparation contains an active content of at least one organophosphorus compound according to one of claims 1 to 7 together with a pharmaceutically acceptable excipient.

12. Pharmaceutical preparation according to claim 11, **characterised in that** the preparation contains another pharmaceutical active substance.

## Revendications

1. Composés organophosphoriques de formule générale (I) : 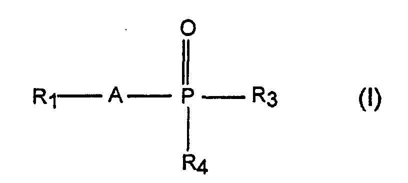 où A est choisi dans le groupe qui consiste en un reste alkyle en C₁₋₉, qui peut présenter une ou plusieurs doubles liaisons et qui peut être substitué par des radicaux hydroxyle, halogène, amino, oxo, avec des radicaux alkyle en C₁₋₉ et des radicaux alcényle en C₂₋₉ linéaires ou non linéaires, où les radicaux alkyle en C₁₋₉ et les radicaux alcényle en C₂₋₉ peuvent'être substitués par hydrogène, des radicaux hydroxyle, amino, halogène et oxo ; -C-O-C- et -C-N-C-, où les atomes de carbone de -C-O-C- et -C-N-C- peuvent être substitués avec un alkyle ayant jusqu'à 7 atomes de carbone ou des radicaux hydroxy ;
ou dans laquelle A correspond à la formule (II) suivante : 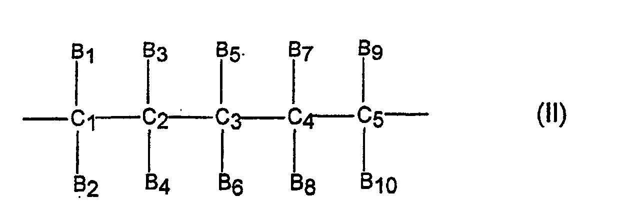 où un ou plusieurs des atomes de carbone, choisis dans le groupe C₃, C₄, C₅, avec leurs substituants peuvent également être omis, et au moins un substituant présent de B₁ à B₁₀ est un radical (cycloalkyl en C₃₋₈)alkyle en C₀₋₉, où non seulement le radical cycloalkyle en C₃₋₈, mais également le radical alkyle en C₀₋₉ peuvent présenter une ou plusieurs doubles liaisons et un ou deux atomes de carbone du radical cycloalkyle peuvent être remplacés par l'atome d'azote, d'oxygène
ou de soufre, et où non seulement le radical cycloalkyle, mais également le radical alkyle peuvent être substitués par des radicaux hydroxyle, halogène, amino, oxo, avec des radicaux alkyle en C₁₋₉ et des radicaux alcényle en C₂₋₉ linéaires ou non linéaires, où les radicaux alkyle en C₁₋₉ et les radicaux alcényle en C₂₋₉ peuvent être substitués par hydrogène, des radicaux hydroxyle, amino, halogène et oxo, et les substituants B₁ à B₁₀ présents restants sont choisis dans le groupe qui consiste en hydrogène, les radicaux hydroxyle, halogène, amino, les restes alkyles en C₁₋₂₆, les restes alkoxy en C₁₋₂₆, les restes (alkoxy en C₁₋₂₆)alkyle en C₁₋₂₆, ou deux substituants d'un atome C forment un radical oxo, où chaque reste alkyle en C₁₋₂₆ et reste alkoxy en C₁₋₂₆ peut être linéaire ou non linéaire et saturé ou insaturé avec une ou plusieurs doubles liaisons et peut être substitué par un radical hydroxyle, amino, halogène ou oxo,
R₁ est choisi dans le groupe qui consiste en les hétérocycles à 5 et 6 membres avec au moins un atome d'azote dans le cycle ou les hydrocarbures polycycliques, qui contiennent au moins l'un de ces hétérocycles, où au moins un de ces atomes d'azote appartient à un radical acide hydroxamique ou ester d'acide hydroxamique, et l'hétérocycle peut être saturé ou insaturé avec une ou plusieurs doubles ou triples liaisons et ainsi, également aromatique et peut être substitué par des radicaux hydroxyle, halogène, amino, oxo, avec des radicaux alkyle en C₁₋₉ et des radicaux alcényle en C₂₋₉ linéaires ou non linéaires, où les radicaux alkyle en C₁₋₉ et les radicaux alcényle en C₂₋₉ peuvent être saturés ou insaturés avec une ou plusieurs doubles ou triples liaisons, et substitués par hydrogène, des radicaux hydroxyle, amino, halogène et oxo, et où l'atome d'azote du radical acide hydroxamique ou ester d'acide hydroxamique est substitué par OR₅, et
R₅ est choisi dans le groupe qui consiste en hydrogène, des radicaux alkyle en C₁₋₉ substitués et non substitués, hydroxyalkyle en C₁₋₉ substitués et non substitués, alcényle en C₁₋₉ substitués et non substitués, alcynyle en C₁₋₉ substitués et non substitués, aryle substitués et non substitués, acyle substitués et non substitués, cycloalkyle substitués et non substitués, aralkyle substitués et non substitués, et des restes hétérocycliques substitués et non substitués,
R₃ et R₄ sont identiques ou différents et sont choisis dans le groupe qui consiste en hydrogène, des radicaux alkyle en C₁₋₂₆ substitués et non substitués, hydroxyalkyle en C₁₋₂₆, aryle substitués et non substitués, acyle substitués et non substitués, aralkyle substitués et non substitués, alcényle en C₁₋₂₆ substitués et non substitués, alcynyle en C₁₋₂₆ substitués et non substitués, cycloalkyle substitués et non substitués, et des restes hétérocycliques substitués et non substitués, halogène, OX₃ et OX₄,
où X₃ et X₄ sont identiques ou différent et sont choisis dans le groupe qui consiste en hydrogène, des radicaux alkyle en C₁₋₂₆ substitués et non substitués, hydroxyalkyle en C₁₋₂₆ substitués et non substitués, aryle substitués et non substitués, aralkyle substitués et non substitués, alcényle en C₁₋₂₆ substitués et non substitués, alcynyle en C₁₋₂₆ substitués et non substitués, cycloalkyle substitués et non substitués, et des restes hétérocycliques substitués et non substitués, un radical silyle, un cation d'une base organique et inorganique, en particulier un métal du premier, deuxième ou troisième groupeus principal du système périodique, ammonium, ammonium substitué et des composés de l'ammonium, qui dérivent de l'éthylènediamine ou d'un acide aminé,
et leurs sels esters et amides et sels d'ester pharmaceutiquement acceptables.

2. Composé selon la revendication 1, **caractérisé en ce qu'**il correspond aux composés organophosphoriques de formule (III) : 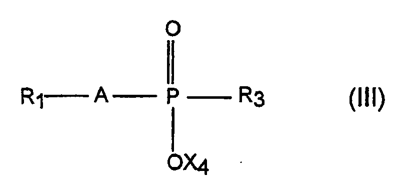 où R₃ est de préférence, hydrogène, méthyle, éthyle ou un reste amide et X₄ est choisi dans le groupe qui consiste en hydrogène, sodium, potassium, méthyle, éthyle.

3. Composé selon la revendication 1, **caractérisé en ce qu'**il correspond aux composés organophosphoriques de formule (IV) : 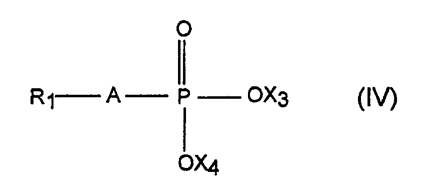 où X₃ et X₄ sont identiques ou différents et sont choisis dans le groupe qui consiste en hydrogène, un radical alkyle en C₁₋₃, un métal du premier, deuxième ou troisième groupe principal du tableau périodique, ammonium, ammonium substitué ou des composés ammonium, qui dérivent de l'éthylènediamine ou d'acides aminés.

4. Composé selon la revendication 3, **caractérisé en ce que** X₃ et X₄ sont identiques ou différents et sont choisis dans le groupe qui consiste en hydrogène, sodium, potassium, méthyle, éthyle.

5. Composé selon l'une quelconque des revendications précédentes, **caractérisé en ce que** A est choisi dans le groupe qui consiste en les radicaux alkylène, alcénylène, hydroxyalkylène et oxoalkylène.

6. Composé selon la revendication 5, **caractérisé en ce que** A est choisi de sorte que entre l'atome d'azote du radical hétérocyclique et l'atome de phosphore, trois atomes sont présents, où A est de préférence un méthylène, hydroxyméthylène, éthylène, éthénylène ou hydroxyéthylène.

7. Composé selon l'une quelconque des revendications 1 à 3, **caractérisé en ce que** le composé est choisi dans le groupe qui consiste en : 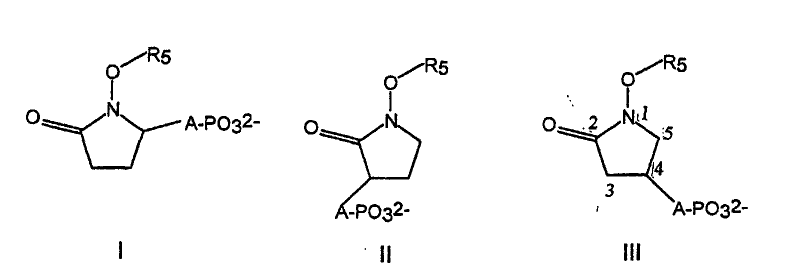 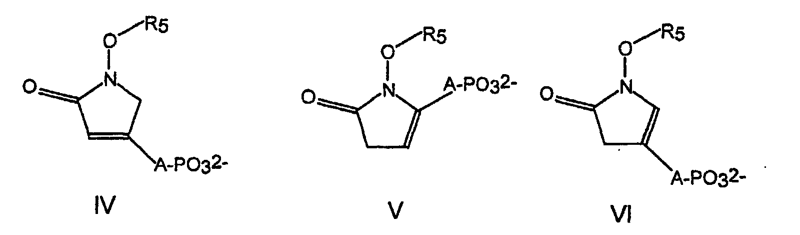 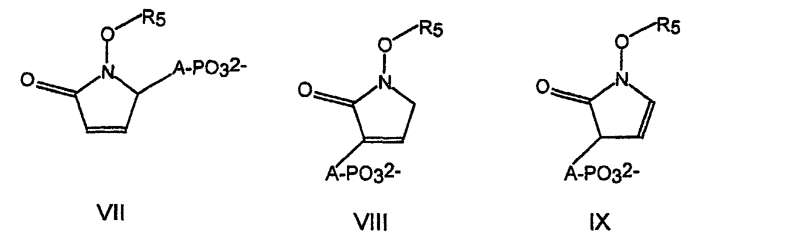 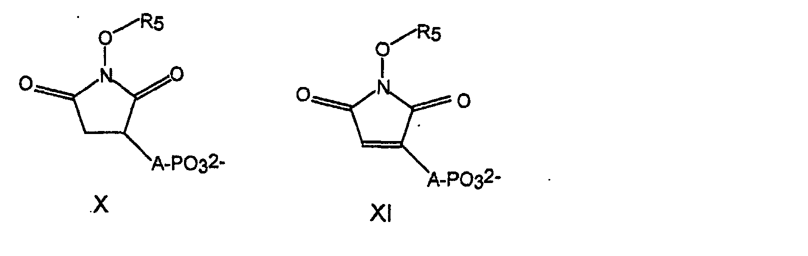 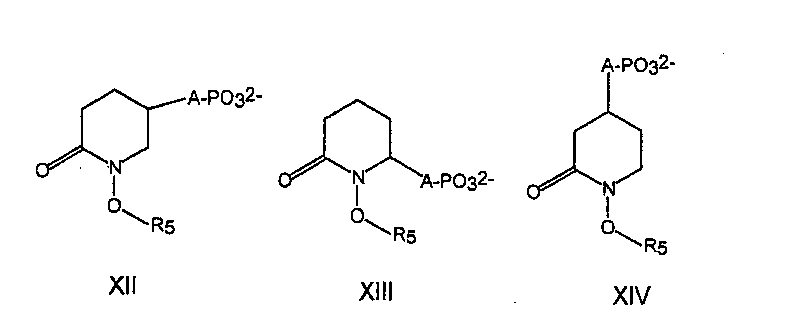 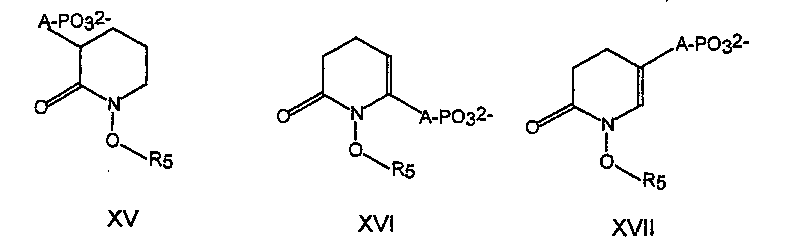 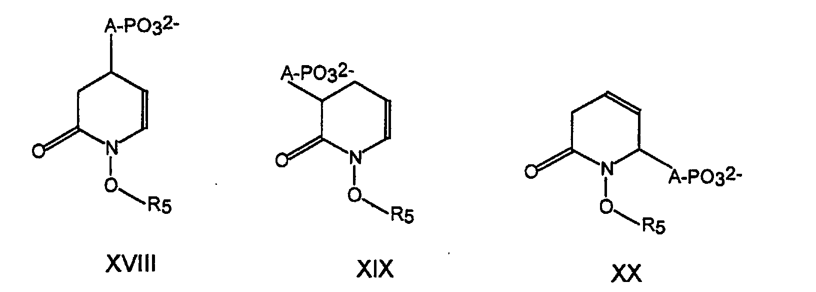 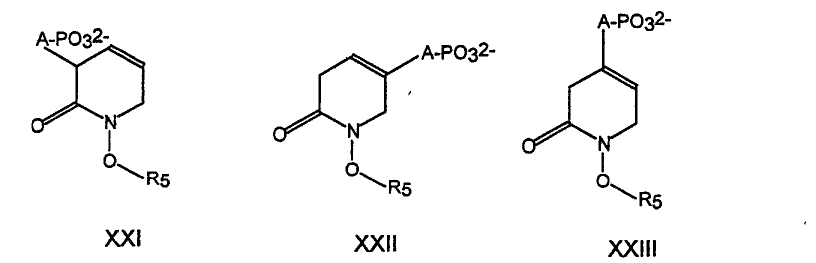 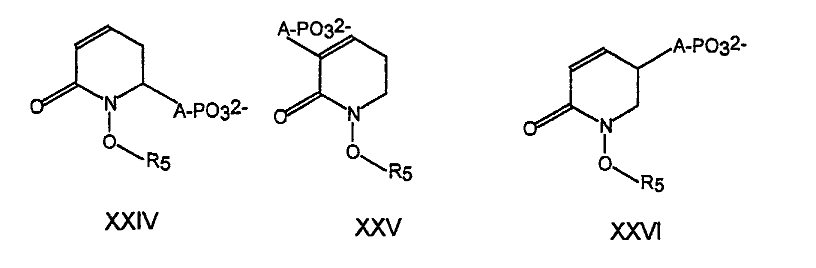 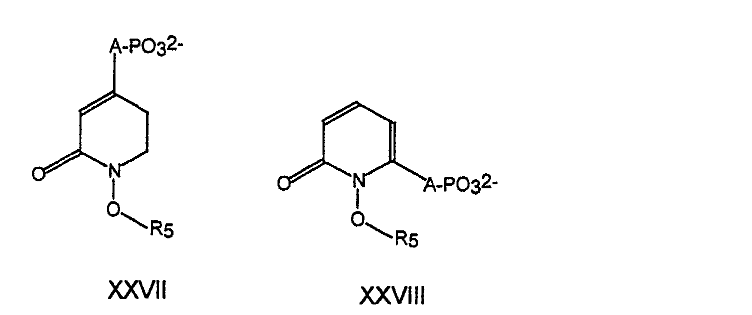 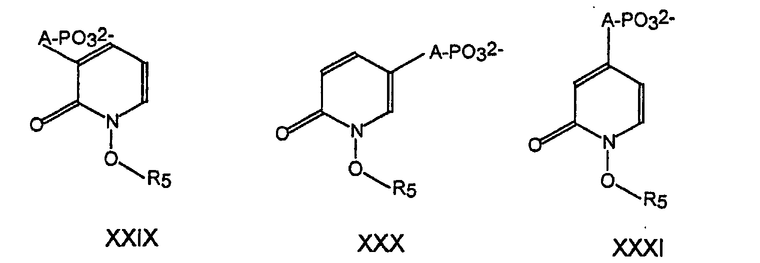 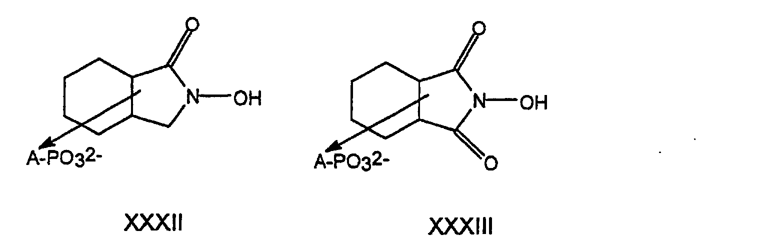 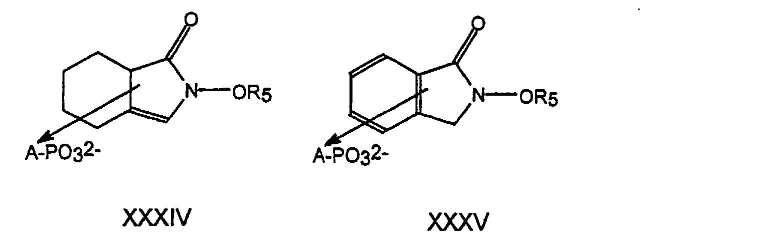 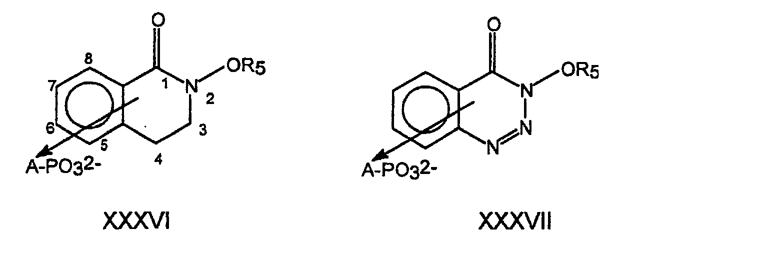 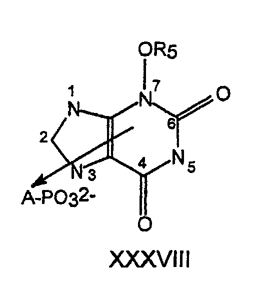 et les dérivés d'acide phosphinique et de phosphinoyle correspondants, où R₅ est défini comme dans la revendication 1.

8. Utilisation d'un composé selon l'une quelconque des revendications 1 à 7, comme fongicide, bactéricide ou herbicide sur des végétaux.

9. Utilisation d'un composé selon l'une quelconque des revendications 1 à 7, pour le traitement d'infections provoquées par des bactéries, des virus, des champignons ou un ou plusieurs parasites.

10. Utilisation selon la revendication 9, pour la prévention ou le traitement d'infections causées par certains parasites, à savoir l'agent pathogène de la malaria, la maladie du sommeil, la maladie de Chagas, la toxoplasmose, la dysenterie amibienne, la leishmaniose, la trichomoniasiose, le pneumocytose, la balantidiose, la kryptosporidiose, la sarkocytose, l'acanthose, la naglerose, la coccidiose, la giardiase et la lambliase.

11. Préparation pharmaceutique pour le traitement thérapeutique et prophylactique de processus infectieux, **caractérisée en ce que** la préparation contient une teneur active en au moins un composé organophosphorique selon l'une quelconque des revendications 1 à 7, avec un support pharmaceutiquement acceptable.

12. Préparation pharmaceutique selon la revendication 11, **caractérisée en ce que** la préparation contient un autre agent actif pharmaceutique.
